# EUROPEAN PATENT SPECIFICATION

(11) **EP 0 555 315 B1**
(45) Date of publication and mention of the grant of the patent: **28.04.2004**
(21) Application number: 91919541.2
(22) Date of filing: 10.10.1991
(51) Int. Cl.: C12N 15/53, C12N 9/02, C12N 5/10, C12Q 1/26, C12Q 1/34, C12N 15/63

(54) **HUMAN CELL LINE STABLY EXPRESSING 5cDNAs ENCODING PROCARCINOGEN-ACTIVATING ENZYMES**
MENSCHLICHE ZELLINIE, DIE STABIL FÜR PROKARZINOGEN-AKTIVIERENDE ENZYME KODIERENDE 5CDNSN EXPRIMIERT
LIGNEE DE CELLULES HUMAINES EXPRIMANT DE MANIERE STABLE DES ADN 5c CODANT DES ENZYMES ACTIVANT DES PROCARCINOGENES

(30) Priority: 15.10.1990 US 597815
(43) Date of publication of application: 18.08.1993
(73) Proprietor: GENTEST CORPORATION, Woburn, MA 01801 (US)
(72) Inventor: CRESPI, Charles, B., Marblehead, MA 01945 (US); PENMAN, Bruce, W., Salem, MA 01970 (US)
(74) Representative: Jump, Timothy John Simon
(86) International application number: PCT/US1991/007487
(87) International publication number: WO 1992/007085

(56) References cited:
- WO-A-87/02984
- ENVIRONMENTAL AND MOLECULAR MUTAGENESIS 1990 , VOL.15, SUPPL 17, page 14 C.L. CRESPI ET AL 'The development of human cell lines which stably express human cytochrome P450 cDNAs' & 21ST ANNUAL MEETING OF THE ENVIRONMENTAL MUTAGEN SOCIETY, ALBUQUERQUE, NEW MEXICO ,USA. MARCH 25-29 1990.
- CHEMICAL RESEARCH IN TOXICOLOGY vol. 4, no. 5 , September 1991 pages 566 - 572 C. L. CRESPI ET AL 'A metabolically competent human cell line expressing five cDNAs encoding procarcinogen-activating enzymes: Application to mutagenicity testing'
- CARCINOGENESIS vol. 11, no. 8 , August 1990 pages 1293 - 1300 C.L. CRESPI ET AL 'Human cytochrome P450IIA3 : cDNA sequence, role of the enzyme in the metabolic activation of promutagens, comparison to nitrosamine activation by human cytochrome P450IIE1'
- MUTATION AND THE ENVIRONMENT vol. 340B , 1990 pages 97 - 106 C.L. CRESPI ET AL 'The development of a panel of human cell lines expressing specific human cytochrome P450 cDNAs' & 5TH INTERNATIONAL CONFERENCE ON ENVIRONMENTAL MUTAGENS July 1989 , CLEVELAND,OHIO, USA.
- MOLECULAR CARCINOGENESIS vol. 3, no. 1 , March 1990 , NEW YORK, US. pages 3 - 8 C.L. CRESPI ET AL 'Stable expression of cytochrome P450IA2 cDNA in a human lymphoblastoid cell line'
- JOURNAL OF BACTERIOLOGY vol. 170, no. 10 , October 1988 pages 4431 - 4436 S. C. SLATER ET AL 'Cloning and expression in Escherichia coli of the Alcaligenes eutrophus H16 poly-beta-Hydroxybutyrate biosynthetic pathway'
- Proceeding National Academy of Science, Volume 82, issued February 1988, I.R. PHILLIPS et al. "Isolation and sequence of human cytochrome P-450 cDNA clone", pages 983-987, see entire article.
- Journal of Biological Chemistry, Volume 261, No. 35, issued 15 December 1986, B.J. SONG et al. "Complementary DNA and protein sequences of ethanol-inducible rat and human cytochrome P-450s", pages 16689-16697, see entire document.
- DNA, Volume 7, No. 2, issued 1988, F.J. GONZALEZ et al., "Human P450 PCNI: Sequence, chromosome localization and direct evidence through cDNA expression that P450 PCNI is Nifedipineousase", pages 79-86, see entire article.
- Carcinogenesis, Volume 10, No. 5, issued 1989, DAVIES et al., "Development of a human cell line by selection and drug-metabolizing gene transfection with increased capacity to activate promutagens", pages 885-891, see entire article.
- Progress in Mutation Research, Volume 5, issued 1985, CRESPI et al., "Tests for mutagenic activity using mutation assays at two loa in human lymphoid cell lines TK6 and AHH-1", pages 497-516, see entire document.
- Science, Volume 209, issued 25 July 1980, BIGGER et al., "Limitations of metabolic activation systems used with in-vitro test for carcinogens", pages 503-504, see entire document.
- Nucleic Acids Research, Volume 14, No. 16, issued 1986, JASWAL et al., "Human P3450: cDNA and complete amino acid sequence", pages 6773-6774, see entire article.

## Description

### Field of the Invention

This invention relates in general to the fields of biochemistry, molecular biology, pharmacology and toxicology, and specifically to a genetically engineered cell line useful in mutagenicity, toxicity and metabolism studies.

### Background of the Invention

The cytochromes P450 are a large family of hemoproteins capable of metabolizing xenobiotics such as drugs, procarcinogens and environmental pollutants as well as endobiotics such as steroids, fatty acids and prostaglandins. Many mammalian cell lines have lost most or all capacity to perform cytochrome P450-catalyzed reactions. This limitation has restricted studies of cytochrome P450 mediated metabolism to either primary cells or tissue homogenates. The lack of adequate endogenous cytochrome P450's has also led to the incorporation of extracellular metabolizing systems, commonly rodent liver homogenates, into assays designed to detect genotoxic effects of promutagens and procarcinogens.

Recently, several laboratories have successfully transfected cytochrome P450 cDNAs into mammalian cell lines. Virtually all of the cell lines were developed by transfecting a single P450 cDNA into the target cell line. While these cell lines may be quite useful for examining P450 specific activation of procarcinogens, given the multiplicity of P450 forms expressed in vivo, it is clear that the use of cell lines expressing single P450s to screen compounds for mutagenic activity will be a daunting task because of the number of cell lines needed. Accordingly, it would be desirable to utilize a small number of cell lines expressing a multiplicity of P450s. It would be even more desirable to have and utilize a single cell line stably expressing all of the human P450 forms primarily responsible for the activation of procarcinogens in human microsomes.

The task of creating a cell line stably expressing a multiplicity of P450s has clear obstacles. Because the P450 genes have substantial homology, it would be expected that a single cell line transfected with multiple P450s would be unstable due to the likelihood of inter or intra vector recombination. Additionally, instability further would be expected in a cell-line containing multiple transfected promoters due to the promoters interfering with one another. These and other obstacles are overcome by the methods and cell lines of the invention.

### Summary of the Invention

According to the invention, a cell line expressing a multiplicity of P450s is provided, as well as methods for creating such cell lines and uses for such cell lines.

Accordingly and in a first aspect, the present invention provides an immortal mammalian cell line stably expressing at least four different transfected cytochrome P450s, at levels below that which renders P450 expression in the cell line unstable, wherein said P450s include P450IA2 and P450IIIA4. Preferably, said P450s include P450IA2, P450IIE1 and P450IIIA4.

In a second aspect, the present invention provides an immortal mammalian cell line stably expressing at least four different transfected cytochrome P450s, at levels below that which renders P450 expression in the cell line unstable, wherein said P450s include P450IIA3 and P450IIIA4. Preferably, said P450s include P450IIA3, P450IIE1 and P450IIIA4.

In preferred embodiments of either aspect of the invention said cytochrome P450s include P450IA2, P450IIA3, P450IIE1 and P450IIIA4.

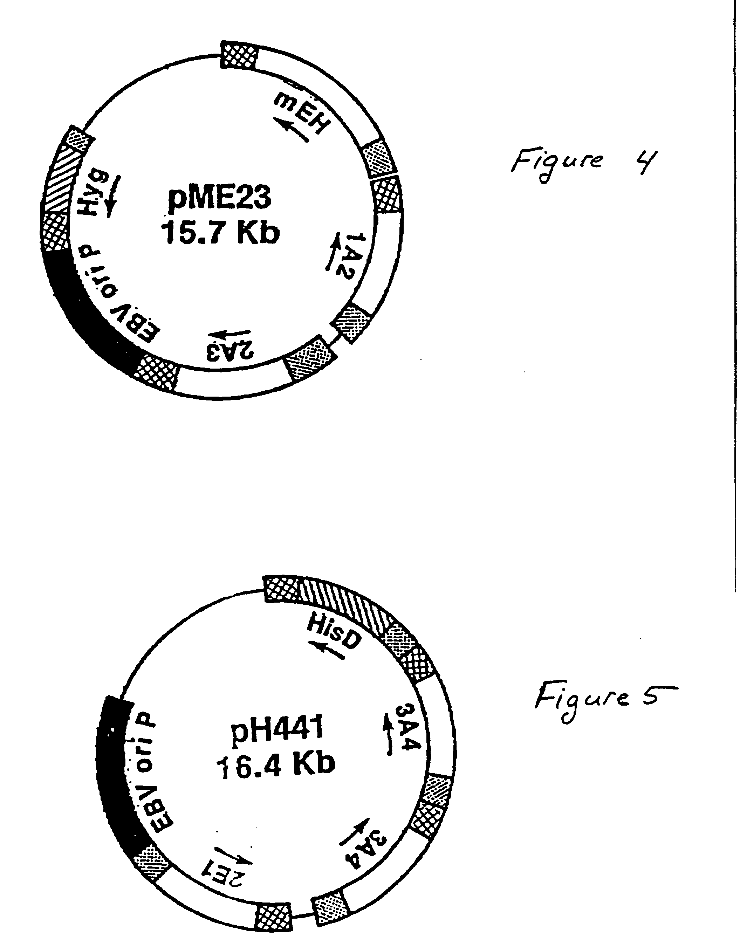

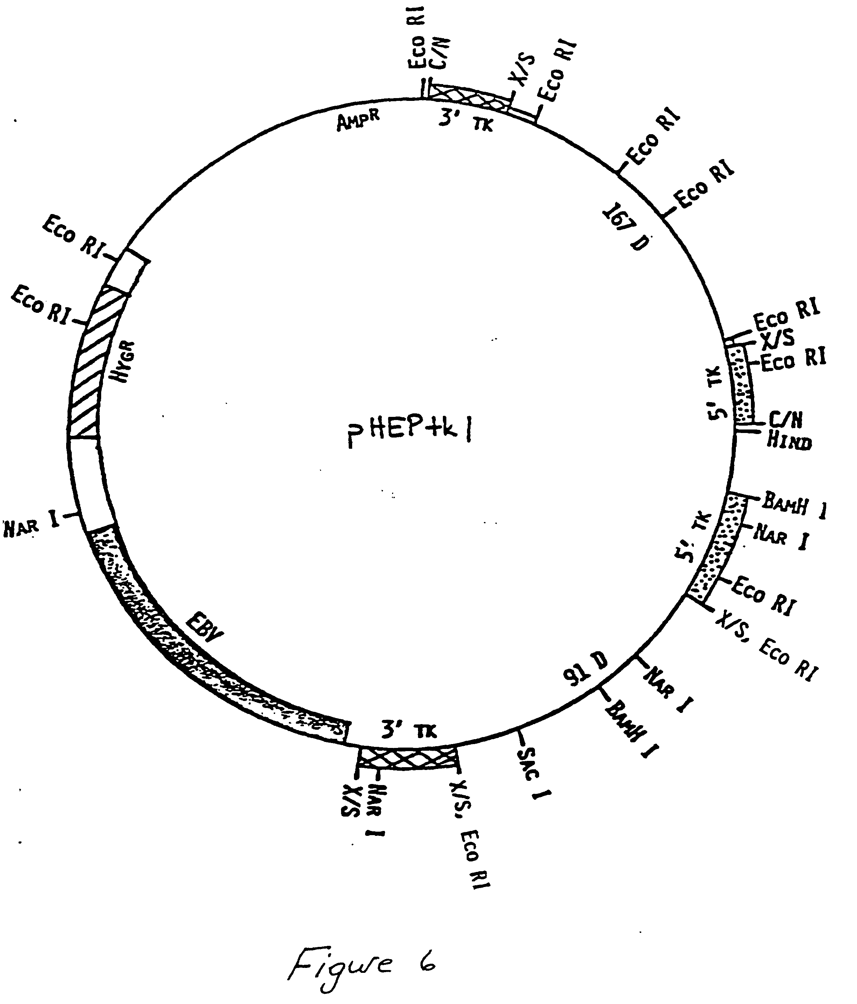

Cell lines in accordance with either aspect of the invention can be transfected with plasmids pME23 as shown in figure 4 and pH441 as shown in figure 5. It is preferred for the cell lines in accordance with the invention to be human cell lines and for all of said cytochrome P450s to be human.

In a third aspect, the present invention provides a method of conducting a metabolism, toxicity or mutagenicity assay, comprising using a cell line in accordance with the first and/or second aspect of the invention. Said method can comprise exposing a culture of a cell line in accordance with the first and/or second aspect of the invention to a test substance and assaying the metabolic, toxic and/or mutagenic effects, if any, of the substance on the cell line.

In preferred embodiments, the inventive method comprises the steps of:
(a) exposing a culture of a cell line in accordance with the first and/or second aspect of the invention to the substance,
(b) detecting the number of mutant cells in the exposed culture, and
(c) comparing the frequency of mutation of the exposed cells to the frequency of mutation of unexposed cells to determine the mutagenicity of the substance.

In contracting state ES, the invention is defined in the attached set of claims identified as being for effect in that state only.

### Brief Description of the Drawings

FIG. 1 is a restriction endonuclease map of a fragment of human DNA including the gene encoding P450IIA3;
FIG. 2 is a restriction endonuclease map of a fragment of human DNA including the gene encoding human epoxide hydrolase;
FIG. 3 is a restriction endonuclease map of the recombinant expression vector pMF6;
FIG. 4 is a restriction endonuclease map of the recombinant expression vector pME23 of the invention;
FIG. 5 is a restriction endonuclease map of the recombinant expression vector PH441 of the invention;
FIG. 6 is a restriction endonuclease map of the expression vector of FIG. 3 including the DNA fragments of both FIG. 1 and FIG. 2;
FIG. 7 is a restriction endonuclease map of the recombinant expression vector of FIG. 3 including the DNA fragment of FIG. 1;
FIG. 8 is a restriction endonuclease map of the recombinant expression vector of FIG. 3 including the DNA fragment of FIG. 2;
FIG. 9 is a table of the enzyme expression levels in the MCL-5 cell line compared to expression in cells having a single cDNA per vector.
FIG. 10 is graph showing the relative survival and sensitivity of the MCL-5 cell line to the mutagenic effects of benzo(a)pyrene, 3-methylcholanthrene and N-nitrosodiethylamine;
FIG. 11 is a graph showing the relative survival and sensitivity of the MCL-5 cell line to the mutagenic effects of aflatoxin B₁; N-nitrosodimethylamine, 2-acetoaminofluorene and benzidine; and
FIG. 12 is a table of the sensitivities of the MCL-5 cell line and AHH-1 cells to the mutagenic effects of benzo(a)pyrene, 3-methylchloranthrene, N-nitrosodiethylamine, N-nitrosodimethylamine, aflatoxin B₁, 2-acetoaminofluorene and benzidine.

### Detailed Description of the Preferred Embodiment

A cell line expressing a multiplicity of P450s is provided according to the invention. The preferred cell line, MCL-5, expresses P450IA2, P450IIA3, P450IIE1, and P450IIIA4, which P450s are those primarily responsible for the activation of the major known procarcinogens. It also expresses P450 IA1, as well as epoxide hydrolase. The cell line thus may be used as a screening tool and avoids the need of using numerous separate cell lines as well as numerous separate assays for screening the known major procarcinogens.

Surprisingly, it was discovered that when P450 expression reaches a certain level (1 pmoles/10⁶ cells for the cell line employed), P450 expression becomes unstable. By unstable it is meant that the activities of the cDNA expressed enzymes change as a function of time of cell growth in culture. Therefore, not only was it necessary to engineer multiple P450s into a single cell line, but it was further necessary to do so in a manner so as to regulate the absolute expression of P450 in the cell and avoid cell shutdown of P450 production. It was anticipated that the use of multiple promoters controlling multiple cDNAs would result in promoter interference and unpredictable or unacceptable expression levels. Likewise, it was anticipated that the relatively high degree of homology between the P450 cDNAs, as well as between their respective promoters, would result in a reduction or loss of expression through recombination. It was also desirable, if not critical, to create a transfection scheme that did not require five separate transfections and five different selectable markers, one each for each transfected cDNA.

Thus, according to the invention, two approaches were simultaneously employed. First, multiple cDNAs were incorporated into single, non-integrating vectors. Second, high expressing cDNAs were incorporated into cell lines at low copy number, whereas relatively low expression cDNAs were incorporated into cell lines at relatively high copy numbers. In this manner, adequate expression of the various P450s was achieved, while maintaining the absolute expression of P450 at a level lower than that which would result in unstable expression of P450s. In the particular cell line used, that level was 1 pmole/ 10⁶ cells. However, it will be understood that the level will vary depending upon the particular cell line chosen, and the invention is not limited to levels of expression below that value. Rather, the invention is intended to encompass cell lines expressing a multiplicity of P450s at levels below that which renders expression unstable, regardless of what the particular, absolute level for a particular cell line may be.

The cell lines according to the invention may be used in mutagenicity assays. For example, a culture of MCL-5 cells is exposed to a substance. Then the exposed cells are grown for a period of time sufficient to allow phenotypic expression. Next, the mutant frequency is determined. Finally, the frequency of mutation of the exposed cells is compared to the frequency of mutation of unexposed cells of the same type to determine the mutagenicity of the substance.

The cell lines may be used in metabolism studies. For example, a culture of MCL-5 cells or an enzyme preparation made from MCL-5 cells are incubated with a test substance. After a period of time the metabolite(s) and unmetabolized test substance are separated, if necessary. Methods of separation may include but are not limited to, solvent extraction, high performance liquid chromatography (HPLC) and other chromatographic separations. The amount of metabolite(s) then is quantified by measuring some physical characteristic of the metabolite(s). Such measurements may include, but are not limited to, absorbence of light, fluorescence and liquid scintillation counting.

The cell lines of the invention also may be used in toxicity assays. For example, a culture of MCL-5 cells is exposed to a substance. Next, the increase in cell number is determined over a period of time. Finally, the increase in cell number of exposed cells is compared to the increase in cell number of unexposed cells to determine the toxicity of the substance. Alternatively, the colony forming ability of exposed cells is compared to the colony forming ability of unexposed cells to determine the toxicity of the substance.

MCL-5 cells were derived from the L3 cell line, a variant of the AHH-1 human B lyphoblastoid cell. Construction of the L3 cell line was described in reference 7. Construction of the parent AHH-1 cell line was described in U.S. Patent 4,532,204. Like the parent cell line, these cells express an enhanced level of P450IA1 activity, grow well in suspension culture, have an infinate life span and have the ability to form colonies in. microtiter plates. Unlike the parent cell line, the MCL-5 cells were found to be more sensitive to the cytotoxicity of benzo(a)pyrene, 3-methylchloranthene, N-nitrosodiethylamine, N-nitrosodimethylamine, Aflatoxin B₁ and 2-acetoaminofluorene.

For all procarcinogens except Benzidine, the MCL-5 cell line was significantly more sensitive than AHH-1 cells. The nitrosamines were more than 10,000 fold more active, Benzo(a)pyrene and Aflatoxin B₁ were about 1000 fold more active and 2-Acetoaminofluorene was 3 fold more active in MCL-5 cells. Only Benzidine gave comparable responses in both cell lines. Based on the mutagenic responses observed in MCL-5 cells and the comparisons to AHH-1 cells, we conclude that we have substantially improved the procarcinogen activating capacity by the transfection procedure. MCL-5 cells were very sensitive to the mutagenic effects of two polycyclic aromatic hydrocarbons, two nitrosamines, a mycotoxin and an aromatic amide. These results indicate that this cell line can activate a spectrum of procarcinogens.

It will become apparent from the following description that immortal human cell lines other than those ultimately derived from AHH-1 could be substituted for the L3 cell line according to various embodiments of the invention.

### Materials and General Procedures

RPMI medium 1640 was obtained from Sigma Chemical Company, St. Louis, MO or Hazelton, Inc., Denver, PA. Horse serum was obtained from Hazelton, Inc.. Cells were propagated in a normal growth medium of RPMI medium 1640 supplemented with 9% v/v horse serum. Cells were cultured in polystyrene tissue culture flasks. Routine cell culturing was performed by determination of cell number by electronic particle counting and diluting the cells with fresh medium to 3 or 4 x 10⁵ cells per ml every day, 2 x 10⁵ cells per ml every two days or 1 x 10⁵ cells per ml every 3 days. Centrifugations of human lymphoblasts were performed at 1000 x g for 5 minutes. Cells bearing recombinant plasmids were maintained in either medium containing 100-200 ug/ml hydromycin B (for pMF6-based vectors) or in medium containing 2 mM 1-histidinol, without 1-histidine (for pEBVHistk-based vectors). Protoplast fusions were performed according to published procedures (1). Plating of the cells was performed in 96 well microtiter plates according to the technique of Furth et al, 1981 (2). Plates were incubated for 13 days to allow colony formation. Hygromycin B was obtained from Calbiochem, La Jolla, CA. Unlabeled and [³H]-benzo(a)pyrene-4,5-oxide were obtained from the National Cancer Institute Chemical Carcinogen Repository. 6-(beta)-hydroxytestosterone standard was obtained from Steraloids, Inc., Wilton, N.H. 6-hydroxychlorzoxaxone standard was a gift of Dr. F.P. Guengerich, Vanderbilt University, Nashville. All other chemicals were obtained from Sigma Chemical Company, St. Louis, MO.

### Libraries and plasmids.

A rat liver cDNA library (RL1001b), a human liver cDNA library (HL1001b) and a human placenta cDNA library (HL1008) were obtained from Clontech, Palo Alto, CA. All three libraries were constructed in bacteriophage lambda gtll (3) and were propagated in E. coli Y1090 (4).

Plasmid pHEBo (5) was obtained from Dr. William Sugden, University of Wisconsin, Madison. The vector pHEBo bears the genes for ampicillin resistance and hygromycin resistance, and contains sequences from the origins of replication of the Epstein-Barr virus and pBR322 which allow it to replicate autonomously in both EBV-transformed lymphoblastoid cells and E. coli (5). pHEBo is 7.1 kb in size and possesses unique sites for the restriction enzymes Cla I, Hind III, Bam HI and Sal I, which may be used for the insertion of the DNA of interest.

Plasmid pHSV106 was purchased from Bethesda Research Laboratories, Gaithersburg, MD. The vector pHSV106 bears the herpes simplex virus thymidine kinase gene (HSV tk gene) and was used as a source for the control sequences of the HSV tk gene. All plasmids were propagated in E. coli HB101 (6).

The construction of the pMF6-based plasmid, designated pHEPtk1, is included in the following pages and was earlier disclosed in pending U.S. patent Application Serial No.07/162,885 and published (7). Plasmid pHEPtk1 contains independently expressed P450IIA3 and microsomal epoxide hydrolase (EH) cDNAs, the gene for hygromycin resistance, and further contains sequences from the origins of replication of the Epstein-Barr virus (EBV) and pBR322 which allow it to replicate autonomously in both EBV-transformed lymphoblastoid cells and E. coli (5). pHEPtk1 is 13.1kb in size and possesses unique sites for the restriction enzyme Hind III, which may be used for the insertion of the DNA of interest.

The constructions of the pEBVHistk vector as well as its derivative plasmids, designated pH44 and pH441, are disclosed in the following pages. The construction of pEBVHistk was earlier disclosed in pending U.S. patent Application Serial No. 07/162,885 and has been reported (8). The construction is reproduced in part herein to facilitate enablement. Plasmid pHBVHistk, contains the E. coli HisD gene which confers 1-histidinol resistance (8) and contains sequences from the origins of replication of the EBV and pBR322 which allow it to replicate autonomously in EBV-transformed lymphoblastoid cells and E.coli. Plasmid pEBVHistk is 9.6 kb in size and possesses unique sited for the restriction enzymes Sal I, Nhe I, and Bam HI, which may be used for the insertion of the DNA of interest. Under appropriate selection conditions, plasmid pHEPtk1 is maintained at 5 copies per diploid cell DNA and pEBVHistk, as well as its derivative plasmids, is maintained at 40 copies per diploid cell DNA.

Restriction endonucleases, the Klenow fragment of DNA polymerase I, and T4 DNA ligase were obtained from New England Biolabs, Beverly, MA and were used according to the manufacturer's recommendations. Calf intestine DNA, alkaline phosphatase and T4 polynucleotide kinase were purchased from Pharmacia, Piscataway, New Jersey. [³²P]- labeled ATP and deoxynucleotides were purchased from Dupont/NEN, Boston, Massachusetts.

### Isolation of the P450IIA3 clone.

The strategy for the isolation of the P450IIA3 clones was as follows. A partial cDNA sequence of a human cytochrome P450IIA3, isolated by hybridization with the rat CDNA encoding P450e, was reported by Phillips and colleagues (9). The cDNA was of sufficient length to encode only two-thirds of the gene. We selected an oligonucleotide at the 5' end of the published sequence to use as a probe of the human liver cDNA library.

The human liver cDNA library HL1001b was plated according to standard procedures (10) at a density of 4.25 x 10⁴ phage per 150 mm petri dish. Plaque replica lifts were performed on Schleicher & Schuell BA-85 nitrocellulose filters according to standard procedures (11). The P450 oligomer (d[pACATTGGAGAATGTGCGGAT]) was end-labeled with polynucleotide kinase and [gamma -³²p] ATP as described (11).

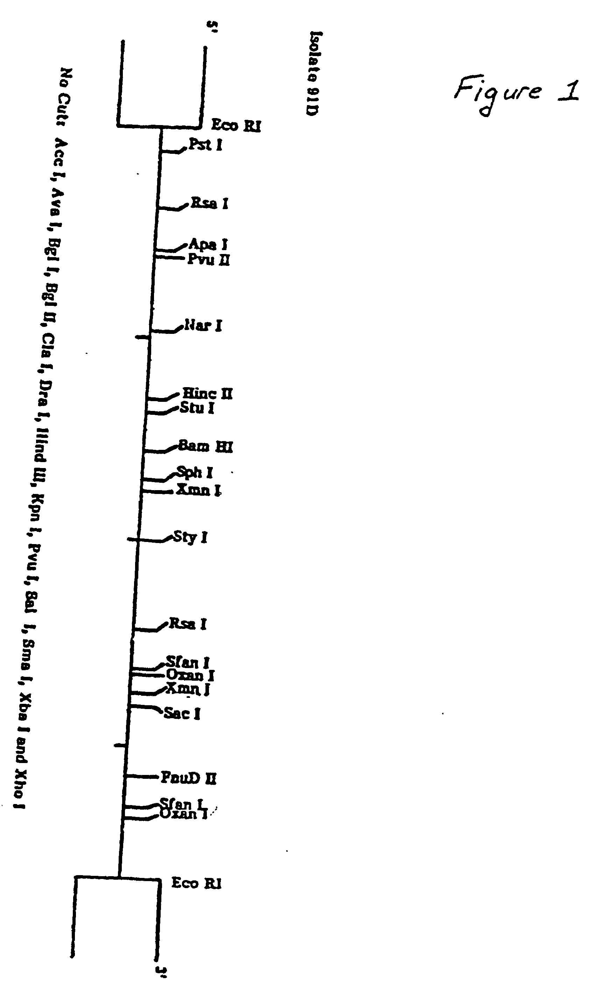

Hybridizations with the P450 oligomer were performed at 30°C in 37.8% formamide, 5x Denhardt's solution (12), 5x SSPE (11), 0.1% sodium dodecyl sulfate (SDS) and 250 ug/ml denatured herring sperm DNA for 16 hours. Following hybridization, the plaque lifts were given three fifteen minute washes at room temperature and three fifteen minute washes at 42°C, each in 500 ml of 2X SSC (13) and 0.1% SDS. The washed filters were then affixed to a sheet of Whatman 3MM filter paper, covered with plastic wrap and exposed to Kodak XAR X-ray film for 1 to 24 hours. Film was developed according to the manufacturer's instructions. Positive plaques were picked using Pasteur pipettes and were purified to homogeneity by successive rounds of plaque replica lifts and creening with the oligonucleotide probe. Of the 125 positive clones, 31 were selected for DNA isolation and analysis. DNA was isolated from purified recombinant phage using the Lambdasorb™ phage adsorbent protocol of Promega, Madison, WI. Lambdasorb™ is a preparation of rabbit polyclonal antibodies directed against bacteriophage lambda particles, in which the antibodies are covalently linked to formalin-fixed Staphylococcus aureus bacterial cells. Bacteriophage lysates, prepared according to standard procedures (11), were incubated with 0.01 volume of Lambdasorb™ for 30 minutes at 0°C. The absorbed phage were precipitated by centrifugation at 7500 RPM in a Beckman Microfuge II for 10 minutes. The precipitated phage were resuspended in 1 ml phage suspension buffer (10 mM Tris-HCL, pH 7.5, 100 mM NaCl, 10 mM MgCl₂, 0.01% gelatin) and centrifuged at 13,000 RPM for 1.5 minutes. The supernatant was discarded. This was was repeated, following which the pallet was resuspended in 0.5 ml 10 mM Tris-HCL pH 7.8, 10 mM EDTA and heated at 70°C for 5 minutes to release the phage DNA from the Lambdasorb™. The preparation was centrifuged at 13,000 RPM for 2 minutes to pellet the absorbent, following which the supernatant was extracted twice with a 1:1 mixture of Tris buffered phenol/chloroform (11) followed by a single extraction with chloroform. The supernatant was then supplemented with 0.25 ml 5 M NaCl and 0.25 ml of 30% polyethylene glycol (of an approximate molecular weight of 8,000 daltons) in 1.5 M NaCl, following which it was mixed well and incubated on ice for 30 minutes. The mixture was centrifuged at 13,000 RPM for 5 minutes, the supernatant was discarded and the DNA pellet was rinsed in 70% ethanol and re-centrifuged. The DNA pellet was dried under vacuum and resuspended in 25 microliters of 10 mM Tris-HCL pH 7.8, 0.1 mM EDTA. The isolated DNA was digested with Eco RI and subjected to electrophoresis on 0.8% agarose gels to determine the size of the cDNA inserts. The gels were stained with 0.5 ug/ml ethidium bromide and bands were visualized under ultraviolet illumination. The clones fell into seven classes based upon insert size of which three were sufficiently large to encode the entire P450IIA3 gene. The 1.8 kb Eco RI fragment of one large clone, designated 91D, was isolated from the agarose gel according to standard protocols (11). (Figure 1) To provide a convenient source of 91D DNA, the fragment was introduced into and propogated in pBR322. The 91D fragment was introduced into the Eco RI site of pBR322 (14), as follows: pBR322 DNA was digested with Eco RI and subsequently treated with calf intestinal alkaline phosphatase to prevent recircularization of the vector. The Eco RI cDNA fragment was combined with the Eco RI-cut PBR322 in three-fold molar excess and ligated into the vector using T4 DNA ligase. The ligated plasmid was then used to transform E. coli HB101, according to the rubidium chloride procedure described in Maniatis et al. (11). Transformed colonies were selected on Luria broth plates supplemented with ampicillin (50 ug/ml). Several colonies were expanded to 5 ml liquid cultures and plasmid DNA was isolated according to the alkaline lysis miniprep procedure described in Maniatis et al. (11). The DNA was digested with Eco RI and subjected to electrophoresis as above to verify the presence of the 1.8 kb band. One such colony was identified and expanded to a 1 liter liquid culture for isolation of milligram quantities of DNA of the recombinant plasmid, p91D. This was performed according to Garger et al. (15). The plasmid DNA was then digested with a series of restriction endonucleases and a detailed restriction map was prepared. Fragment 91D was compared at corresponding regions with the fragment described by Phillips et al. (9). Fragment 91D shares sites for the following restriction enzymes with the fragment described by Phillips et al: Bam HI, Hinc II, OxaN I, Rsa I, Sac I, SfaN I, Sph I, Stu I, Sty I and Xmn I. A conflict was observed in the position of the site for the restriction enzyme FnuD II, which could represent a sequence difference due to allelic variation. Based on the similarities in the restriction maps we have assigned the 91D isolate to the P450IIA3 class.

The location of the 5' end of 91D was determined by digestion with restriction enzyme Bam HI followed by agarose gel electrophoresis and Southern blotting (13). The blots were probed with the 5' oligomer and the assignment of the location of the 5' end was based upon the pattern of hybridization.

### Isolation of the Epoxide Hydrolase clone.

The strategy for the isolation of the human epoxide hydrolase (EH) gene required two parts, as follows: The gene for the rat EH has been isolated (16) and sequenced (17) and could therefore be used as a probe of a human cDNA library in order to isolate the human gene. An oligonucleotide was prepared from the published sequence and was used to isolate a rat EH clone, designated 14A.

The rat liver cDNA library RL1001b was plated at a density of 4.3 x 10⁴ phage per 150 mm plate and nitrocellulose replica lifts were performed. The oligomer (d[pCCCCAGTCCCCGCCTTGAAT]) was end-labeled with [gamma-^{32p}] ATP as above. Hybridizations with the rat epoxide hydrolase oligomer were performed at 30°C in 50% formamide, 5x Denhardt's solution, 5x SSPE, 0.1% SDS and 250 ug/ml denatured herring sperm DNA. After hybridizing for 16 hours, the filters were subjected to three 15 minute washes at room temperature, two 15 minute washes at 42°C and one 15 minute wash at 52°C, each in 2X SSC and 0.1% SDS. XAR X-ray film was exposed and developed as described above. Positive plaques were picked using Pasteur pipettes and were purified to homogeneity by successive rounds of screening. DNA was isolated from recombinant phage as above using the Lambdasorb™ protocol of Promega, Madison, WI, and was digested with Eco RI to determine the size of the cDNA inserts. The cDNAs fell into seven classes which ranged in size from 0.74 kb to 1.6 kb. A representative of the largest class (clone 14A, 1.6 kb) was selected for further analysis.

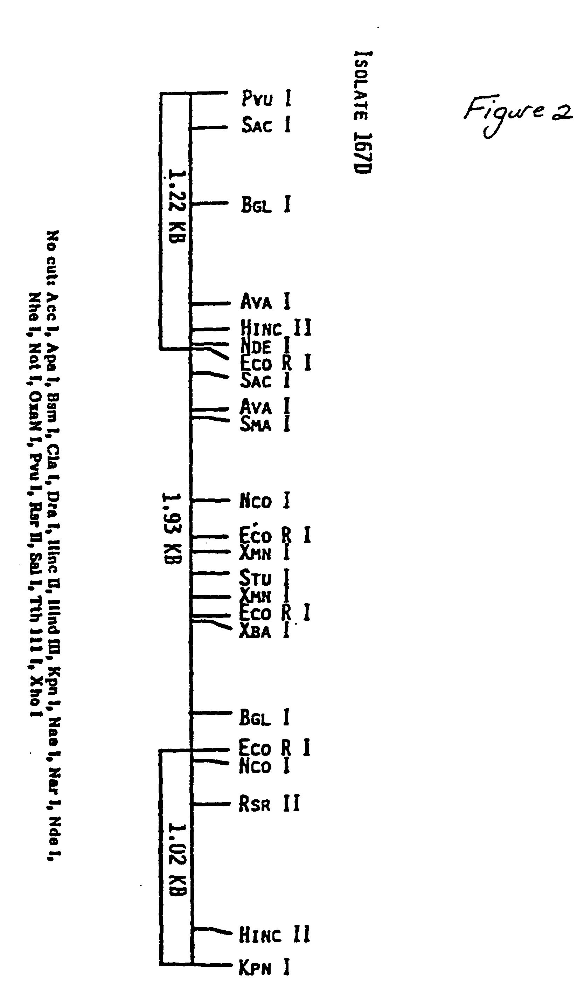

The identity of 14A was verified by extensive restriction mapping and comparison with a computer-generated map of the published rat EH sequence. The 1.6 kb Eco RI fragment of 14A was subcloned into the Eco RI site of pBR322, resulting in the plasmid p14A. A 5' Eco RI-Bam HI fragment of p14A was used to probe the human liver and placenta cDNA libraries, HL1001b and HL1008. HL1001b was plated at a density of 4.3 x 10⁴ plaques per 150 mm plate and HL1008 was plated at a density of 5 x 10⁴ plaques per plate. The 5' Eco RI-Bam HI fragment of p14A (0.46 kb) was nick translated to a specific activity of 5 x 10⁷ cpm/ug with [alpha-³²P] dCTP and TTP (11), and the plaque lifts were probed with the fragment in 50% formaldehyde hybridization solution as above. The plaque lifts were given three 15 minute washes at room temperature followed by one 30 minute wash at 68°C, each in 2X SSC and 0.1% SDS. A total of 42 positive clones were identified, of which 36 were found in the liver library and six were found in the placenta library. DNA from 14 clones was isolated. Restriction analysis determined that the clones fell into eight classes based upon size. The largest clone, 167D, was of sufficient length (1.93 kb) to encode the entire EH gene. (Figure 2) The presence of two internal Eco RI sites necessitated the use of alternate restriction enzymes in the subcloning of the sequence. Clone 167D was excised with Kpn I and Pvu I which resulted in the subcloning of 2.24 kb of phage DNA flanking the cDNA. The overhanging 3' ends were rendered blunt-ended with Klenow fragment of E. coli DNA polymerase I and Hind III linkers were kinased and ligated to the fragment (11). The 4.17 kb fragment was inserted into the Hind III site of pBR322. Following transformation of HB101, isolation of DNA from transformed colonies and digestion with Hind III to ascertain the presence of the 4.17 kb 167D fragment, a plasmid designated p167D was selected. Plasmid DNA was isolated and a detailed restriction map was prepared. As was described above for clone 91D, the determination of the 5' end of clone 167D was achieved by Southern hybridization of an Eco RI restriction digest with the 5' Eco RI-Bam HI fragment of the rat EH clone 14A.

### The P450IA2 clone.

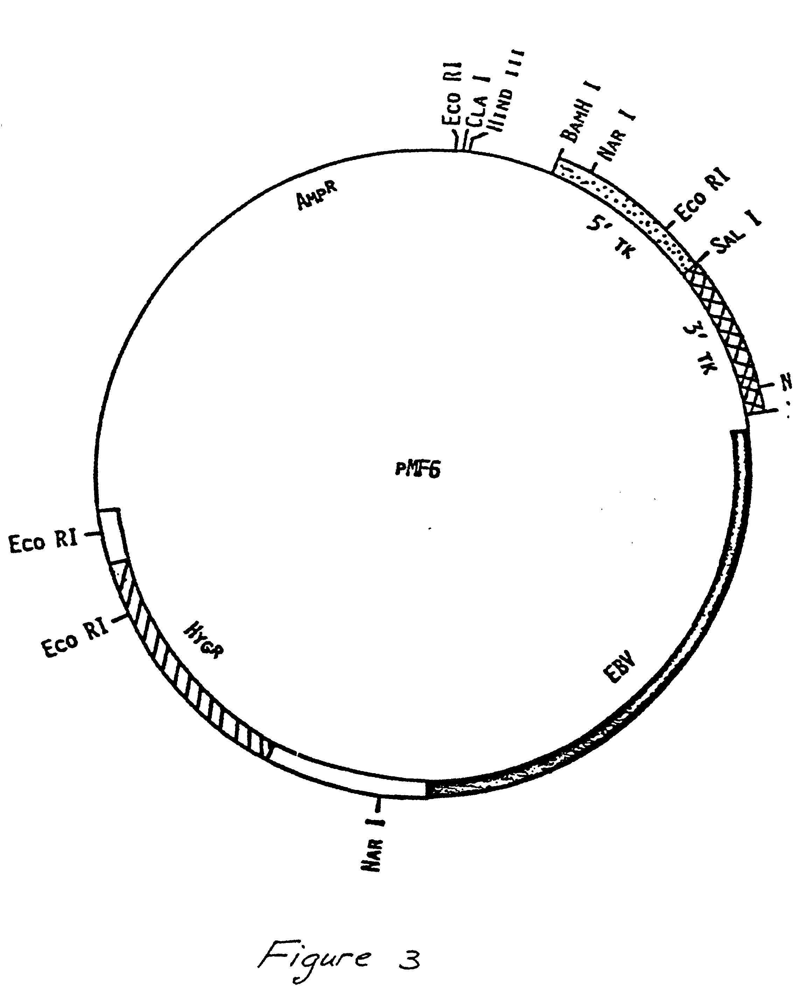

The P450IA2 cDNA (in the ECO RI site of pUC9) was originally obtained from Drs. Frank Gonzalez and Harry Gelboin at the National Cancer Institute. cDNA was isolated from the recombinant plasmid by digestion with Eco RI and Dral, to yield a 1.6-kb fragment containing the entire coding sequence. The restriction enzyme-digested sticky ends were rendered blunt-ended with the Klenow fragment of E. coli DNA Polymerase I and Xhol linkers were ligated to the fragment.(11) The modified cDNA was introduced into the unique Sal I site of the expression vector pMF6. (Figure 3) Following transformation of HB101, isolation of DNA from transformed colonies and analysis of structure by restriction mapping, the plasmid was designated pMF6/IA2 Partial. The DNA was isolated from the plasmid and was subjected to partial digestion with Nar I to isolate and purify the 2.6 kb fragment containing the P450IA2 cDNA, the promoter and polyadenylation signal of HSVtk.

### The P450IIIA4 clone.

The isolation of the P450IIIA4 clone has been published (19). This clone was originally obtained from Drs. Frank Gonzalez and Harry Gelboin at the National Cancer Institute. cDNA was isolated from the recombinant plasmid by digestion with Bsu 36 I and partial digestion with Eco RI, to yield a 1.6 kb fragment (19) containing the entire coding sequence. The restriction enzyme-digested fragment was rendered blunt-ended and modified by the addition of Xho I linkers and introduced into the unique Sal I site of the pMF6 expression vector (20). This vector was designated pMF6/3A4.
A 2.6 kb Nar I fragment containing the P450IIIA4 cDNA, the promoter and polyadenylation signal of HSVtk was isolated and purified.

### Isolation of the P450IIE1 clone.

The human cytochrome P450IIE1 cDNA was isolated using an oligonucleotide probe whose sequence was based on the published sequence for human P450IIE1 cDNA (21). The oligonucleotide was complementary to basepairs 82 to 101 in the coding sequence of the cDNA and had a sequence of 5'GCAGCTGGAATCTGCCCC 3'. The oligonucleotide was end labeled with ³²P and used to screen a human liver cDNA library in λgt11 (HL1001b).

The human liver cDNA library HL1001b was plated according to standard procedures (10) at a density of 4.25 x 10⁴ phage per 150 mm petri dish. Plaque replica lifts were performed on Schleicher & Schuell BA-85 nitrocellulose filters according to standard procedures (11). The P450 oligomer (d[pACATTGGAGAATGTGCGGAT]) was end-labeled with polynucleotide kinase and [gamma -³²P] ATP as described (11).

Hybridizations with the P450 oligomer were performed at 30°C in 37.8% formamide, 5x Denhardt's solution (12), 5x SSPE (11), 0.1% sodium dodecyl sulfate (SDS) and 250 ug/ml denatured herring sperm DNA for 16 hours. Following hybridization, the plaque lifts were given three fifteen minute washes at room temperature and three fifteen minute washes at 42°C, each in 500 ml of 2X SSC (13) and 0.1% SDS. The washed filters were then affixed to a sheet of Whatman 3MM filter paper, covered with plastic wrap and exposed to Kodak XAR X-ray film for 1 to 24 hours. Film was developed according to the manufacturer's instructions. Positive plaques were picked using Pasteur pipettes and were purified to homogeneity by successive rounds of plaque replica lifts and screening with the oligonucleotide probe. Positive colonies were purified, the cDNA inserts were excised, Lambdasorb™ subcloned into the Eco RI site pUC19 and restriction maps were generated. The restriction map of the largest isolate, designated 257A, showed considerable agreement with that of the published P450IIE1 cDNA (8).

In order to determine whether the cDNA isolate contained the complete coding sequence, 100 basepairs at the 5' end were directly sequenced in pUC19. Isolate 257A was found to be 11 basepairs short of the complete coding sequence. There were also 3 base substitutions in the isolate relative to that of Song et al (21), all of which were cryptic. A cDNA with the complete coding sequence was generated by blunt-end ligation of synthetic oligonucleotides. Isolate 257A was cut at the 5' cloning linker (Eco RI) and at the 3' end with Nde I. The DNA fragment was rendered blunt-ended with Mung Bean nuclease, and the synthetic oligonucleotides shown below were ligated onto the blunt-ended fragment.

Xho I Start
5¹ TCGAG'ATGTCTGCCG 3¹
3¹ C TACAGACGGC 5¹

These oligonucleotides were synthesized to correspond to the published 5' cDNA coding sequence and to have Xho I "sticky ends" to facilitate subcloning into the expression vector pMF6 (8). Ligation of the oligonucloetides onto the blunt-ended cDNA resulted in a sequence containing a single, conservative amino acid substitution at position 4 (alanine for leucine). This substitution is not expected to affect enzyme function. The ligated mixture was digested with Xho I and the desired fragment was purified and subcloned into the unique Sal I site in the expression vector pMF6. The correct construct was designated p257Atk2. A 2.6 kb NarI fragment containing the P450IIEI cDNA, promoter and polyadenylation signal for HVtk was isolated and purified according to standard procedures.

### CONSTRUCTION OF pME23 AND pM441 EXPRESSION VECTORS

The following sections describe the construction of the pME23 and pH441 expression vectors which were used to transfect L3 cells. Plasmid pME23 contains P450IA2, P450IIA3 and mEH cDNAs. pME23 is derived from pHEPtk1, a pMF6-based plasmid containing the P450IIA3 and P450EH genes. Plasmid pH441 is derived from plasmid pH44, a pEBVHIStk-based plasmid, and contains two P450IIIA4 cDNAs and one P450IIEl cDNA.

Schematic Maps of the pME23 and pH441 Vectors are shown in Figures 4 and 5, respectively. The thin line represents pBR322 sequences, the black box represents the Ori P sequences derived from EBV, the hatched boxes represent the hygromycin B resistance gene (Figure 4) or the E. Coli His D gene (Figure 5), the stippled boxes represent the HSV promoters, the cross hatched boxes represent the HSV polyadenylation signals and the open boxes represent the cDNAs. Identities of the cDNAs and direction of transcription are indicated on the figures.

### A. Construction of Expression Vector pME23

### A1. Construction of Expression Vector pHEPtk1.

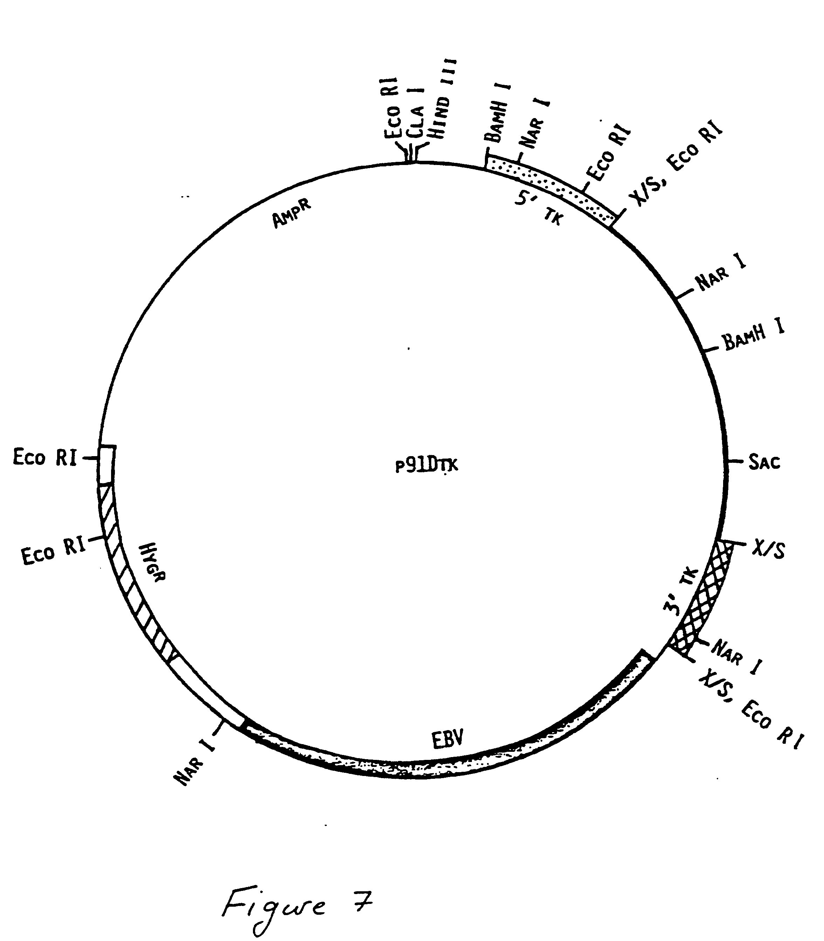

Plasmid pHEPtk1 (Figure 6) contains transcriptional units for P450IIA3 and EH. The construction of this vector was as follows:

As determined by the restriction analyses, neither of the clones P450IIA3 nor EH was cut by Sal I or Xho I. Thus, constructing an expression vector having a promoter sequence and a poly A addition signal sequence separated by a unique Sal I site would allow easy insertion of the cloned fragments into the expression vector. The plasmid pHEBo has a unique Sal I site, and this plasmid was selected as the starting point for creating the expression vector with control regions separated by a unique Sal I site.

The expression vector for the P450IIA3 and EH genes was created as follows: Plasmid pHSV106 (17), bearing the Herpes simplex virus thymidine kinase gene, was digested with Pvu II, and then Xho I linkers were added (11). The DNA was then digested with Sma I and Sal I linkers were attached to create fragments with Xho linkers at one end and Sal I linkers at the other end. Included was a 0.6 kb fragment bearing the 3' poly A addition signals of the HSV tk gene (22, 23), which could now be inserted into the Sal I site of pHEBo.

pHEBo was digested with Sal I and subsequently treated with calf intestinal alkaline phosphatase to prevent recircularization of pHEBo during the ligation step. The 0.6 kb fragments with Xho linkers at one end and Sal I linkers at the other were added and the DNA was ligated. Since both Xho linkers and Sal linkers will ligate to the Sal I cut in the pHEBo, two orientations of the fragment were possible. Of the two orientations, the fragment would be correctly inserted when the preserved Sal I site at the 5' end of the fragment was proximal to the Bam HI site of pHEBo, and the nonfunctional 3' Sal I-Xho I fusion site was distal to the Bam HI site. Following ligation, bacteria were transformed with the newly created plasmids and plasmid DNA was isolated from transformed colonies. Isolated plasmid DNA from different colonies was digested with Eco RI and with both Cla I and Sal I to determine the presence and orientation of the inserted HSV tk sequences. The plasmid having the correct orientation was designated p12L.

The 5' promoter sequences were isolated from pHSV106 by cutting the plasmid with Bgl II, filling in the ends with the Klenow fragment of E. coli DNA polymerase I (11) and adding Sal I linkers. The DNA was then cut with Bam HI to create a 0.7 kb fragment having a Bam HI cut site at the 5' end and a Sal I linker at the other 3' end. Then, plasmid p12L was cut with Bam HI and Sal I, the 0.7 kb fragment was added to the p12L cut DNA and the total DNA was ligated and used to transform HB101. DNA then was isolated from transformed HB101 colonies utilizing the miniprep procedure, and the DNA was digested with Pst I, Eco RI and both Bam HI and Sal I to identify the correct construction. The plasmid so obtained, designated pMF6, contained both 5' and 3' controlling sequences from the HSV tk gene and preserved the uniqueness of the Sal I site for use in the insertion of genes to be expressed in the human lymphoblasts.

### A2. Introduction of the P450IIA3 cDNA into the Expression Vector pMF6.

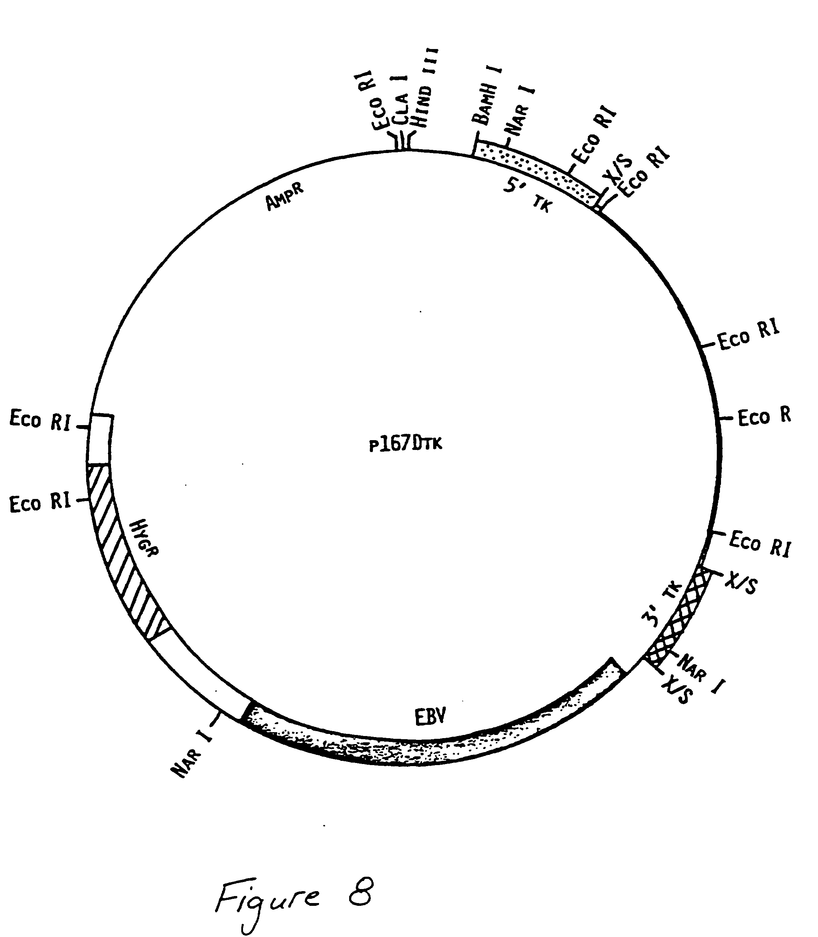

Plasmid pMF6 DNA was digested with Sal I and treated with calf intestine phosphatase, as above. The P450IIA3 clone 91D was excised from plasmid p91D with Eco RI. The resulting fragment was made blunt-ended using the Klenow polymerase fragment and Xho I linkers were added, following which the fragment was ligated directly into the Sal I-cut pMF6. HB101 was transformed with the ligated plasmid, DNA was isolated from the resulting colonies and the orientation of the fragments relative to the HSV tk sequences was.determined by digestion with Bam HI. The appropriate construction was designated p91Dtk. (Figure 7)

### A3. Introduction of the EH cDNA into the Expression Vector pMF6.

The introduction of the EH clone into pMF6 was complicated by the presence of internal Eco RI sites in the clone. Extensive restriction mapping of plasmid p167D revealed that use of Nde I and Rsr II would allow excision of clone 167D with only 220 bp of flanking phage DNA (less than 50 bp at the 5' end and approximately 180 bp at the 3' end). The resulting fragment of 2.15 kb was rendered blunt-ended with Klenow and Xho I linkers were attached. The fragment was then ligated into Sal I-cut pMF6. DNA was isolated from transformed HB101 colonies and a recombinant plasmid bearing the EH clone in the appropriate orientation relative to the HSV tk sequences was identified by a double digestion with the restriction enzymes Hind III and Xba I. This plasmid was designated p167Dtk. (Figure 8)

### A4. Consolidation of P450IIA3 and EH cDNAs on a Single pMF6 Plasmid.

In order to simplify introduction of the novel activities into the improved lymphoblastoid cell line L3, a plasmid carrying both of the newly isolated genes together with their attendant control sequences was constructed. The entire expression region of p167Dtk, including the necessary HSV sequences at both the 5' and 3' ends, was excised with Nar I. The resulting 3.2 kb fragment was introduced into the unique Cla I site of p91Dtk. The 167Dtk sequences can be ligated into the p91Dtk plasmid in either of two possible orientations, both of which may be expected to be productive. Bacteria were transformed with the ligation mixture and DNA was isolated from transformed colonies using the alkaline lysis miniprep procedure. The orientation of the inserted DNA was determined by a double digestion with Hind III and Xba I, and plasmids with inserts in both orientations were obtained. In pHEPtk1, the 167Dtk sequences will be transcribed in the opposite direction from the 91Dtk sequences. In the alternate plasmid, designated pHEPtk2, the two genes are transcribed in the same direction.

### A5. Introduction of the P450IA2 cDNA into Vector pHEPtk1 to Construct Expression Vector pME23.

Plasmid pHEPtk1 was digested with Hind III, the cut was rendered blunt ended and modified by the addition of Cla I linkers and then treated with phosphatase. The vector was ligated to the 2.6 kb Nar I fragment obtained from partial digestion of pMF6/IA2 (18) which contains the complete P450IA2 transcriptional unit described earlier.

HB101 was transformed with the ligated plasmid, DNA was isolated from the resulting colonies and the identity of the construct was verified by restriction mapping. The resulting construction was designated pME23. (Figure 4)

### B. Construction of Expression Vector pH441.

The following sections describe construction of the pEBVHIStk-based expression vector, pH441, having the cDNAs for P450IIIA4 and P450IIE1.

### B1. Construction of Expression Vector pEBVHistk.

pEBVHistk was constructed as follows: the 2.5 kb Nar I fragment of pMF6 containing the EBV origin of DNA replication was introduced into Nar I digested pBR322 (Short HSV tk sequences at either end of this fragment not functional) and the construct was designated pEBV. Isolation of the 2.5 kb Nar I fragment was earlier disclosed in pending U.S. Application Serial No. 07/162,885, a summary of which is reproduced below to facilitate enablement. The 2.5 kb Nar I fragment of pMF6 containing the E. coli HisD gene (Cut with Ava II at the 5' end and Hind III at the 3' end, approximately 600bp at the 3' end were removed with Ba131 nuclease) was introduced into the Cla I site of pEBV to generate pEBVHis. Finally, the 1.0 kb Nar I fragment of pMF6 was modified by the addition of Hind III linkers and introduced into the Hind III site of pEBVHis to generate pEBVHistk.

### B2. Introduction of the P450IIIA4 Gene into Vector pEBVHistk to Construct Expression Vector pH44.

Plasmid pH44 was constructed as follows: the 1.6 kb Eco RI/Bsu36 I fragment of the P450IIIA4 cDNA (19) containing the entire coding sequence was modified by the addition of Xho I linkers. The modified cDNA was introduced into the unique Sal I site of the pMF6 expression vector (20). This vector, designated pMF6/3A4, was digested with Nar I and a 2.6kb Nar I fragment containing the entire P450IIIA4 transcriptional unit (described above) was isolated and ligated into the pEBVHis vector (24) after modifying the Hind III site by the addition of Cla I linkers and treating with calf intestine phosphatase. This vector confers resistances to 1-histidinol and is maintained at high copy number (40 per cell). In order to partially compensate for the anticipated lower expression of P450IIIA4, a plasmid containing 2 complete P450IIIA4 transcriptional units was constructed and designated pH44. To facilitate this construction in a single step, a 10:1 molar ratio of P450IIIA4-containing 2.6 kb fragment to vector was used in the ligation.

### B3. Introduction of the P450IIE1 cDNA into Vector pH44 to Construct Expression Vector pH441.

Plasmid pH44 was subjected to a Nar I partial digest and calf intestine phosphatase treatment. The vector was ligated to the 2.6 kb Nar I fragment of p257Atk2 (8) which contains a complete P450IIE1 transcriptional unit as described above. The identity of the construct was verified by restriction mapping and the resulting plasmid was designated pH441. (Figure 5)

### TRANSFECTION PROCEDURES

### A. Transfer of Plasmid pH441 into L3 cells.

Plasmids pH441 and pME23 were introduced sequentially into the L3 cell line via protoplast fusion. Plasmid pH441 was introduced first.

Plasmid pH441 was introduced into L3 cells using protoplast fusion (12). E. coli HB 101 containing the plasmid pH441 were grown in Luria broth containing 50 ug/ml ampicillin (150 ml total volume). When the bacteria concentration yielded a OD₆₀₀ of 0.6, 200 ug/ml chloramphenicol was added to the culture and the culture was stirred overnight. Bacteria were centrifuged 2000 x g for 10 minutes and the cell pellet resuspended in 1.5 ml of HBS-20 (20 mM HEPES, 20% sucrose, pH 7.1) and 0.48 ml of a 10 mg/ml lysozyme solution (in HBS-20, filter sterilized) was added. The bacteria were incubated at room temperature for 45 minutes. The lysozyme reaction was terminated by the addition of 0.24 ml of 1.25 M CaCl₂. Excess Ca⁺⁺ was chelated by adding 0.6 ml of 0.25 M EDTA. The above protoplast preparation was diluted by the addition of 6 ml of HBS-9 (20 mM HEPES, 9% sucrose, pH 7.1).

L3 cells (2 to 3 x 10⁷ cells) were centrifuged and the cell pellet dispersed by tapping the tube. 1.5 ml of PEG-fusion reagent (PEG-fusion reagent consisted of 48% (w/w) polyethylene glycol 1450 purified according to (36) with the balance RPMI medium 1640) was added to the cells. Unless otherwise specified, a reference to normal medium in this protocol indicates RPMI medium 1640, supplemented with 9% Horse serum. 2.5 ml of the protoplast suspension was added immediately and the resulting suspension was centrifuged at 800 x g for 3 minutes. The pellet was dispersed by tapping the tube, 1.5 ml PEG-fusion reagent was added and the cells were incubated for 1 minute. The PEG-fusion reagent was diluted with 50 ml of normal medium, the cells were centrifuged and resuspended in 80 ml of normal medium containing 100 U/ml penicillin, 100 ug/ml streptomycin and 100 ug/ml gentamycin (PSG). One day after protoplast fusion, 2.6 x 10⁷ cells were centrifuged (1000 x g for 5 min.) and resuspended in medium containing 0.5 mM 1-Histidinol (without 1-histidine) and PSG as described above. Five days after protoplast fusion the culture was diluted 50 ml to 80ml with the same medium. Eight days after protoplast fusion 50 ml of the culture was centrifuged, resuspended in 20 ml of the same medium and added back to the culture. Eleven days after protoplast fusion the culture was diluted by the addition of 30 ml medium containing 2 mM Histidinol. From this point forward the cell concentration was determined every 2 to 5 days and when it exceeded 2.5 x 10⁵ cells/ml the culture was diluted (without 1-Histidine) and PSG. Twenty three days after protoplast fusion the culture was diluted 58 to 100 ml by the addition medium containing 2 mM 1-Histidinol (without 1-histidine) and PSG. Twenty nine days after protoplast fusion the culture was diluted to 1.5 x 10⁵ cells/ml in 100 ml with the same medium. Thirty three and thirty seven days after protoplast fusion the culture was again diluted as described above. Forty and forty two days after protoplast fusion, the culture was diluted to 2 x 10⁵ cell/ml in 100 ml with the same media. Forty four days after protoplast fusion, the culture was diluted to 1 x 10⁵ cells/ml in 100 ml with the same media. Forty seven days after protoplast fusion, the cells were plated in 96 well microtiter plates in the above media. Cells were aliquotted over 8 plates at an average of 0.5 cells per well. The plates were incubated for 12 days. Colonies were isolated and scaled-up in media-containing 2 mM 1-histidinol (without 1-histidine) to bulk cultures (approximately 100ml).

P450IIIA4 (as measured as testosterone 6(B) hydroxylase activity), P450IIE1 (as measured as sensitivity to the cytotoxicity of N-nitrosodimethylamine), and native P450IAl (as measured as 7-ethoxy-resorufin deethylase activity) enzymatic activities and the structure and copy number (via southern Blotting of Eco RI digested genomic DNA) of the vector were verified in the clon.al populations. One clonal population was chosen for subsequent transfection. This cell line containing approximately 40 copies of the pH441 vector, exhibited a testosterone 6-(B) hydroxylase activity of 1.5-1.9pmole product/10⁶cells min. Exposure to 100 ng/ml N-nitrosodimethylamine reduced survival to 0.18 relative to control (untreated) cells and exhibited 7-ethoxyresorufin deethylase activity (after pretreatment for 20 hrs with 0.1 uM dibenz(a,h)-anthracene) of 1.6 pmole product/10⁶ cells-min.

It should be understood that the above protocol for protoplast fusion represents an actual embodiment of the invention and that the exact number of days between cell dilution may vary as a function of cell growth rates. Cell dilution is indicated when cells have doubled in number in comparison to the previous dilution. Accordingly, cell dilution is indicated when the cell count has exceeded between approximately 4 x 10⁵ and 6 x 10⁵ cells per ml.

### B. Transfer of Plasmid pME23 into L3 cells previously transfected with Plasmid pH441.

The same protoplast fusion procedure described above in connection with the transfer of plasmid pH441 into an L3 cell line was employed for the insertion of plasmid pME23 into the pH441-transfected L3 cell line with the following exceptions:

Selection was based on resistance to both 2mM 1-histidinol and hygromycin B. Following transfection, cells were cultured in media containing 2 mM 1-Histidinol (without 1-histidine), 30ug/ml 4-aminolevulinic acid, 100 ug/ml penicillin, 100 ug/ml streptomycin and 100 ug/ml gentamycin. One day after protoplast fusion cells were diluted to 50 ml at 2.5 x 10⁵ cells per ml and 400 ug/ml hygromycin B was added. Five days after protoplast fusion the cells were diluted 50 ml to 80 ml and 300 ug/ml Hygromycin B was added. Eight days after protoplast fusion 50 ml of the culture was centrifuged, resuspended in 20 ml of fresh medium, added back to the culture and 200 ug/ml hygromycin B was added to the culture. Eleven days after protoplast fusion the bulk population began to grow; cells were diluted to 2 x 10⁵ cells per ml (80 ml total volume). Thereafter cells were either diluted to 2 x 10⁵ cells per ml and 100 ug/ml hygromycin B was added every 2 days or cells were diluted to 1 x 10⁵ cells per ml and 200 ug/ml hygromycin B added every 3 days. The bulk transfected population was designated MCL-5.

### ASSAYS.

### A. Enzyme Assays.

Enzyme activities were measured in whole cells. Coumarin 7-hydroxylase was measured according to the method of Greenlee and Poland (25) by adding 100 uM coumarin directly to the cell cultures and measuring fluorescence production after a two hour incubation. Microsomal epoxide hydrolase was measured according to the procedure of Glatt et al. (26) using 5 x 10⁶ cells in a 0.5 ml total volume and incubating for 30 minutes. 7-ethoxyresorufin deethylase activity (27) was measured in whole cells according to (28). All other enzyme assays utilized 1 x 10⁷ cells in a 1 ml incubation volume. Acetanilide 4-hydroxylase was measured according to (29). Chlorzoxazone 6-hydroxylase was measured according to (30). Testosterone hydroxylase was measured according to Waxman et al (31).

### B. Cytotoxicity and Mutagenicity Assays

Cytotoxicity was estimated by measuring growth after exposure to the procarcinogen. After cultures have resumed exponential growth, the cumulative growth of the mutagen-treated cultures was divided by the cumulative growth of the negative control cultures to yield relative survival.

Stock cultures of MCL-5 cells were propagated in RPMI 1640 medium (without histidine) supplemented with 9% horse serum, 100-200 ug/ml hygromycin B and 2mM 1-histidinol and 30 ug/ml 4-aminolevulinic acid as described above. Before use in a mutagenesis assay aliquots of stock MCL-5 cells were grown for three days in HAT medium (stock culture medium containing 100 uM hypoxanthine, 0.8 uM aminopterin, 35 uM thymidine), centrifuged, resuspended in TH medium (HAT without aminopterin) and grown for three days with dilutions by stock culture medium. The HAT/TH treatment removes pre-existing tk and hgprt mutants.

Aliquots of MCL-5 cells (3x10⁷) cells in 60 ml) were exposed to one or more concentrations of the test chemical, to solvent alone and to a known mutagen, usually in replicate cultures, for 28 hr. Mutagen exposure was performed on normal medium containing 50-100 ug/ml hygromycin B, and 1-2 mM 1-histidinol and 15 to 30 ug/ml 4-aminolevulinic acid. Chemical exposure was terminated by centrifuging the cultures and resuspending the cells in fresh medium (usually 3x10⁷ cells in 80 ml) without hygromycin or 1-histidinol. Cultures were then passaged (generally every other day to 3x10⁷ cells in 150 ml) to allow phenotypic expression of induced mutation (at least three days for the tk locus and at least seven days for the hgprt locus). After sufficient time to allow phenotypic expression of induced mutation, aliquots of the cultures were plated in 96 well microtiter plates to determine the mutant fraction. Cells were plated at an average of 2.5x10⁴ cells per well in the presence of 4 ug/ml trifluorothymidine (usually three plates) to measure mutation at the tk locus, at an average of 2.5x10⁴ cells per well (usually three plates) in the presence of 0.6 ug/ml 6-thioguanine to measure mutation at the hgprt locus and at an average of 2.5 cells per well (usually two plates) without selection to measure the plating efficiency. The plates were incubated for 13 days and scored for the presence or absence of a colony in each well. The calculation of mutant fraction and the statistical analysis were as described previously (32).

### EXAMPLES

### Example 1: Enzyme Expression in MCL-5 Cells and Comparison to Expression with a Single cDNA per Vector.

Given the multiplicity of related vectors and the large number of Herpes Simples virus (HSV) tk gene-derived promoters and polyadenylation signals (4 per vector), the stability of the cell line and the efficiency of expression of each cDNA were concerns.

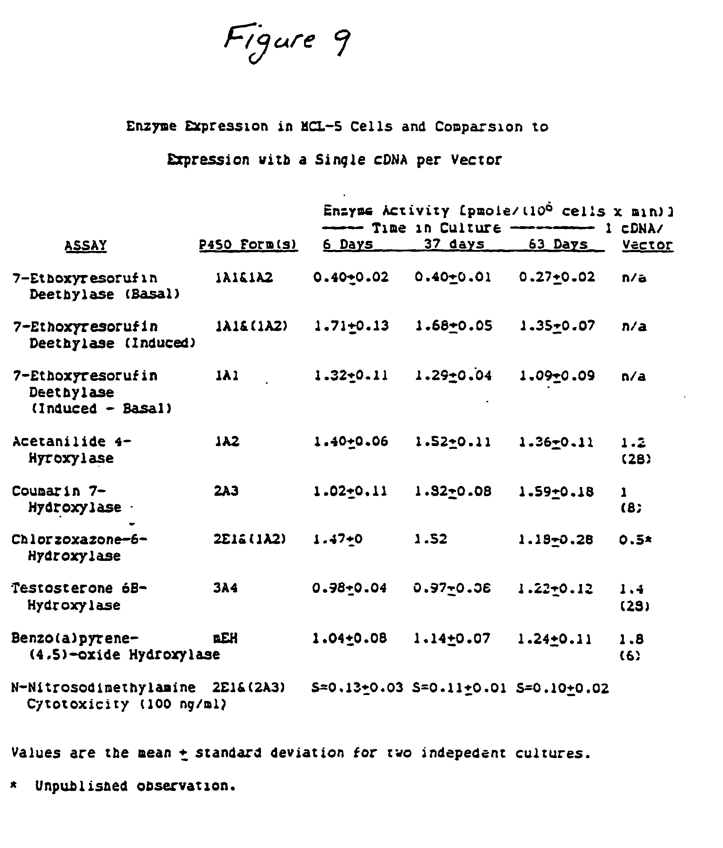

To verify cDNA and native P450IA1 expression and also to examine stability of the cell line, a series of cytochrome P450 form-specific enzyme assays and procarcingen activations were measured as a function of time in cell culture (Figure 9). The levels of the activities in MCL-5 cells were compared to those observed in cells transfected with a single cDNA with the same means of selection.

The levels of induced 7-ethoxyresorufin deethylase activity in MCL-5 cells were comparable to those observed in the untransfected L3 cell line (7). Acetanilide 4-hydroxylase activity (P450IA2 specific), testosterone 6(beta)-hydroxylase (P450IIIA4-specific) and microsomal epoxide hydrolase activity in MCL-5 cells were comparable (within a factor of 2) to those observed in cells transfected with pMF6/IA2 (18), p91Dtk (pMF6/2A3; (33)), pH44 (pEBVHistk with two CYP3A4 cDNAs;) and p167Dtk (pMF6/mEH;(7)) respectively. The levels of chlorzoxazone 6-hydroxylase (P450IIE1-specific) in MCL-5 cells were about 3 fold higher than in cells bearing pEBVHis/2E1. Based on these assays and comparisons, all cDNAs as well as the native P450IAl were adequately expressed. It appears that there was no substantial promoter interference in these vector constructs.

It should be noted that although higher levels of stable expression for each of the above cDNAs have been achieved in constructs containing a single cDNA, this invention achieves expression of a multiplicity of P450 forms. There is certainly an upper limit to the P450 content (presently unknown) which will be stably supported in this cell line. These constructions were designed such that the total P450 content would be no more than that previously observed to be stably supported in this system (e.g. cells bearing P450IIA3 in the pEBVHistk vector contain about 1 pmole P450 per million cells).

This was achieved by placing two inefficiently expressed P450 cDNAs (P450IIIA4 and P4502E1) in the high copy number vector (pEBVHistk) and two efficiently expressed P450 cDNAs in the low copy number vector (pMF6). Thus the total anticipated P450 content was kept below 1 pmole/million cells.

The stability of expression at the enzyme level was determined by monitoring the levels of the enzyme activities as a function of time of growth in cell culture. For this experiment we initially cultured the transfected cells and scaled-up the size of the culture sufficiently to establish a freezer stock of several hundred vials. The zero time point represented establishing a culture from this stock. Independent vials were thawed at different times points and were assayed simultaneously in cultures that were propagated for 6, 37 and 63 days (Figure 9).

The levels of enzyme activities did not vary substantially as a function of time in culture. The maximum difference among two time paints was less than a factor of 1.5. Analysis over a two month time frame indicates that the MCL-5 cell line is sufficiently stable to permit many applications including long-term, low-dose exposure experiments (34,35) and transfer of the cell line to independent laboratories.

Based on P450-specific enzyme assays, we conclude that MCL-5 cells simultaneously express the human P450 forms which have been established to be primarily responsible for the activation of many procarcinogens. Therefore MCL-5 cells have the potential for be an effective screening tool for human procarcinogen activation.

### Example 2: Procarcinogen Activation by MCL-5 Cells: Cytotoxicity and Mutagenicity Assay Procedures.

The following procedure is directed to cytotoxicity and mutagenicity assays following exposure of the MCL-5 cells to the procarcinogen benzo(a)pyrene. The identical procedure was used to assess cytotoxicity and mutagenicity for each of the procarcinogens tested.

Mutant fractions were determined at the hprt locus for all procarcinogen concentrations and were also determined at the tk locus for the highest procarcinogen concentration. The results are presented in the following examples. In general, the negative control mutant fractions at the hprt and tk loci in MCL-5 cells were comparable to those observed in the parent L3 cell line. This indicated that the expression of the cytochrome P450s was not substantially affecting the spontaneous mutation rate (possibly through the generation of activated oxygen species). This observation is consistent with our previous observations that P450 expression does not affect background mutant frequency (38-41).

In general, there was good agreement between hprt and tk in the magnitude of the mutagenic response observed. Values were all within a factor of 2. This indicates that the tk locus can be used for many screening applications with a significant savings in time and materials because of the shorter phenotypic expression time at tk relative to hprt.

Cytotoxicity was estimated by measuring growth after treatment. After cultures have resumed exponential growth, the cumulative growth of the mutagen-treated cultures was divided by the cumulative growth of the negative control cultures to yield relative survival. Induction of mutation at the hprt and tk loci was measured by previously published protocols (32) with minor modifications. Human lymphoblasts were exposed to the mutagen for 28 hours. Each replicate culture contained 3x10⁷ cells. After a 7 day phenotypic expression period, the mutant fraction was measured according to the procedures described above. Aflatoxin B₁, Benzo(a)pyrene, 3-Methylchloranthrene, 2-Acetoaminofluorene and Benzidine were dissolved in dimethylsulfoxide; N-Nitrosodimethylamine and N-Nitrosodiethylamine were dissolved in water for delivery to the cell cultures.

### Example 3: Toxicity and Mutagenicity of Benzo(a)pyrene

The toxicity and mutagenicity of the carcinogen benzo(a)pyrene was determined using the procedure described above. Benzo(a)-pyrene is known to be metabolized by the P450IA1 to mutagenic species. This mutagenicity can be further potentiated by the action of epoxide hydrolase in concert with the P450IA1 to generate a vicinal diol-epoxide.

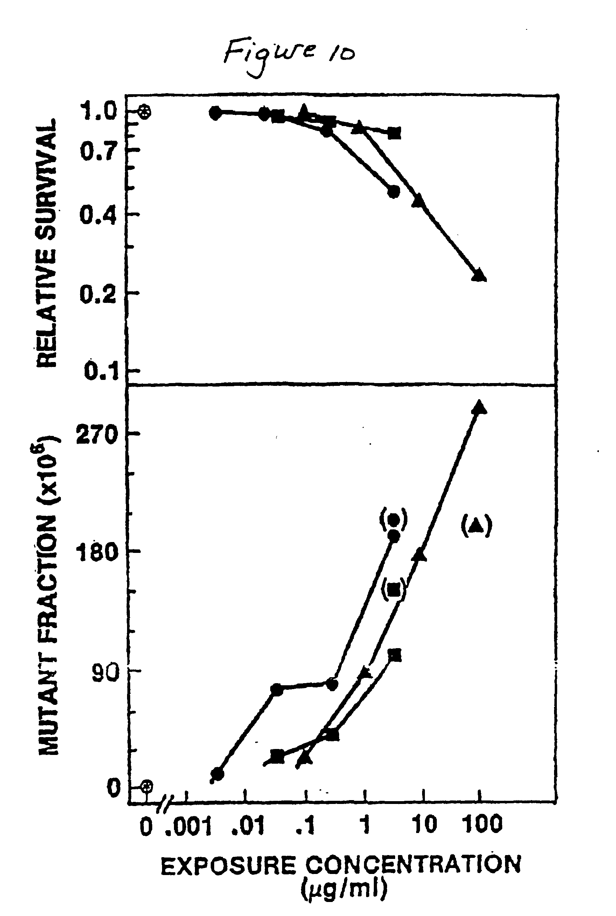

The mutagenicity of benzo(a)pyrene, 3-methycholanthrene and N-nitrosodiethylamine was determined at the hprt and tk loci in MCL-5 cells. Procarcinogen cytotoxicity and mutagenicity is illustrated in Figure 10. Cells were exposed to the indicated concentrations of the mutagens for 28 hours. Each chemical was tested in at least two independent experiments with at least duplicate cultures in each experiment. Mean values are plotted. All concentrations were assayed for mutant fraction at the hprt locus, the highest concentration was also assayed for mutant fraction at the tk locus. The tk locus data points are enclosed in parentheses. Procarcinogens are as follows: circles, benzo(a)pyrene; squares, 3-methycholanthrene and triangles, N-nitrosodiethylamine. The mean negative control mutant fractions were 2.8x10⁻⁶ and 3.6x10⁻⁶ at the hprt and tk loci respectively.

Benzo(a)pyrene induced a significant mutagenic response at an exposure concentration of 3 ng/ml and monotonic increase in mutant fraction was observed over a 1000 fold concentration range (Figure 10). Benzo(a)pyrene was not particularly cytotoxic; substantial increases in mutant fraction were observed in the absence of significant cytotoxicity and exposure to 3 ug/ml only reduced relative survival to 60%.

MCL-5 cells were 3 to 10 fold more sensitive to Benzo(a)pyrene than MCL-1 cells which express P450IIA3 and mEH under hygromycin B selection (7). This observation is consistent with P450IA1 and mEH being primarily involved in this activation and suggests a potential role for other P450's in the activation of benzo(a)pyrene. Shimada, et al. (36) have reported that P450IIIA4 can activate Benzo(a)pyrene-7,8-diol in human liver microsomes.

### Example 4: Toxicity and Mutagenicity of N-nitrosodiethylamine

N-nitrosodiethylamine cytotoxicity and mutagenicity were determined in MCL-5 cells (Figure 10).

N-Nitrosodiethylamine induced a significant mutagenic response at an exposure concentration of 100 ng/ml, and like Benzo(a)pyrene, a monotonic increase in mutant fraction was observed over a 1000 fold concentration range including non-cytotoxic exposure concentrations (Figure 10). We have previously demonstrated that both P450IIA3 and P450IIE1 can activate N-Nitrosodiethylamine (33). The mutant fractions observed in MCL-5 cells was about 2 fold higher than predicted based on the sum of the mutant fractions in IIA3/Hyg cells and IIE1/Hol cells (33). The higher mutant fractions in MCL-5 cells may be due to the apparently higher levels of P4502E1 as measured by chlarzoxazone 6-hydroxylase activity and N-Nitrosodimethylamine sensitivity.

### Example 5: Toxicity and Mutagenicity of N-nitrosodimethylamine

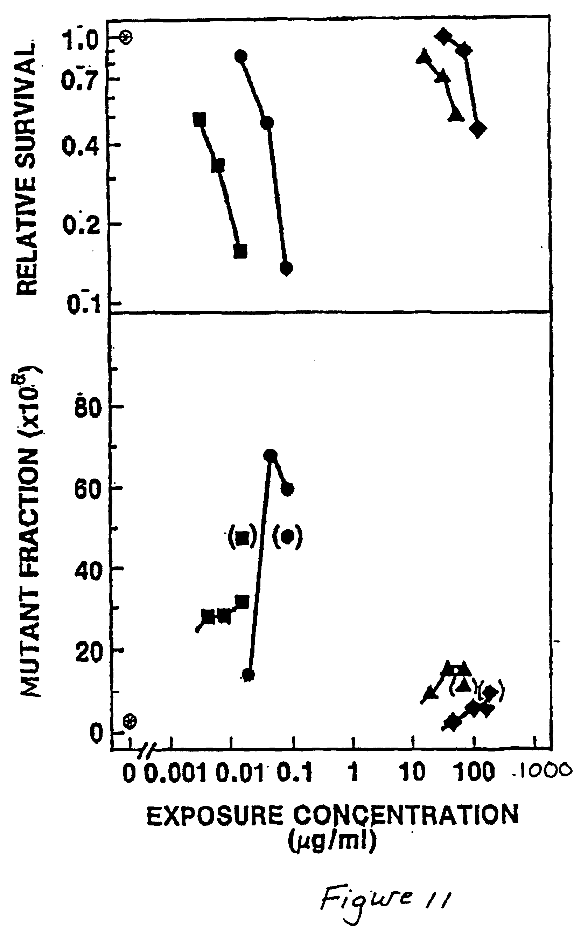

N-nitrosodimethylamine cytotoxicity and mutagenicity were determined in MCL-5 cells (Figure 11). Procarcinogens are indicated in Figure 11 as follows: circles, N-nitrosodimethylamine; squares, Aflatoxin B₁ and triangles, 2-acetoaminofluorene and diamonds, benzidine. The experiments were performed and results plotted as described above.

N-Nitrosodimethylamine induced a significant increase in mutant fraction at an exposure concentration of 20 ng/ml (Figure 11). This chemical was also quite cytotoxic. MCL-5 cells were about 3 fold more sensitive to N-nitrosodimethylamine than 2El/Hol cells (33) which is consistent with the higher levels of chlorzoxazone 6-hydroxylase activity in MCL-5 cells. P450IIA3 plays a minor role in the activation of N-Nitrosodimethylamine (1000 fold less active than P450IIE1) which probably did not significantly contribute to the increased mutagenic response.

### Example 6. Toxicity and Mutagenicity of Aflatoxin-B1 at the tk and hgprt Loci

The toxicity and mutagenicity of the carcinogen aflatoxin B₁ was examined using the procedures described above. Aflatoxin-B₁ is metabolized by several cytochrome P450 enzymes including the P450IA1, P450IIA3, P450IIIA4 and P450IA2. Therefore it was anticipated that MCL-5 cells would be more sensitive to Aflatoxin B₁ induced mutagenicity.

Aflatoxin B₁ induced a significant increase in mutant fraction at an exposure concentration of 5 ng/ml (Figure 11). Unlike Benzo(a)pyrene, 3-Methylchloranthrene and N-Nitrosodiethylamine, Aflatoxin B₁ was quite cytotoxic at low concentrations. We have previously observed Aflatoxin B₁ to be activated to a mutagen in 1A2/Hyg, 3A4/Hol and 2A3/Hyg cells (unpublished results) with 1A2/hyg cells being about 8 fold more sensitive than 3A4/Hol cells which were about 15 fold more sensitive than 2A3/Hyg cells. The native cell line is also sensitive to Aflatoxin B₁. The sensitivity of MCL-5 cells was consistent with the sum of individual enzymatic activation capacities.

### Example 7: Toxicity and Mutagenicity of 3-Methylchloranthrene

3-Methylchloranthrene induced a significant increase in mutant fraction at an exposure-concentration of 30 ng/ml and a monotonic increase in mutant fraction was observed over a 100 fold concentration range (Figure 10). Again, substantial increases in mutant fraction were observed in the absence of significant cytotoxicity.

The mutant fractions observed in MCL-5 cells for Benzo(a)pyrene, N-nitrosodiethylamine and 3-Methylchloranthrene were among the highest observed in this system and were greater than many direct-acting alkylating agents. This observation underscores the substantial procarcinogen activating capacity in MCL-5 cells.

### Example 8: Toxicity and Mutagenicity of 2-Acetoaminofluorene

2-Acetoaminofluorene has been reported to be activated by P450IA1 and P450IA2 (37).
2-Acetoaminofluorene induced a significant increase in mutant fraction at an exposure concentration of 10 ug/ml as illustrated in Figure 11. The response in MCL-5 cells was greater than that observed in the parent AHH-1 cell line and thus was consistent with one of the transfected and higher native P450IA1 activities mediating the activation. Further testing with cell lines expressing single cDNAs will be required to establish which form(s) were responsible.

### Example 9: Toxicity and Mutagenicity of Benzidine

Benzidine induced a very modest increase in mutant fraction (2 to 3 fold, Figure 11). The response in MCL-5 cells was similar to that observed in parent AHH-1 cell line (discussed further below). Indicating that metabolism by the transfected activities was probably not the rate limiting step for the activation of this compound to a mutagen.

### Example 10: Comparison of MCL-5 Cell Line Sensitivity To Parent AHH-1 Cell Line

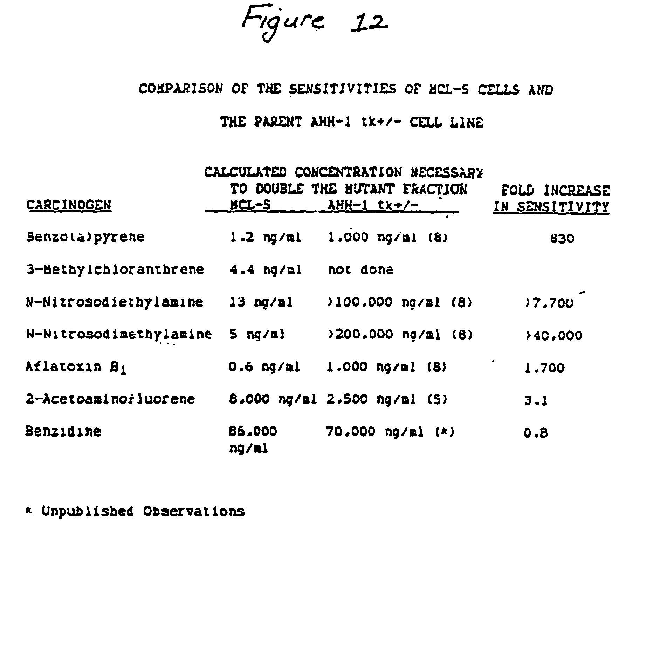

In order to further illustrate the effects of cDNA transfection, the sensitivity of the MCL-5 cell line was compared to that of the parent AHH-1 cell line which contains only native P4501A1 activity. For this comparison, the concentrations of mutagens necessary to yield a doubling in mutant fraction at the hprt locus were calculated by linear interpolation. A doubling in mutant fraction represents a significant increase in mutant fraction. This comparison was possible for all chemicals except 3-Methylchloranthrene for which data was not available on the AHH-1 cell line. Both AHH-1 and MCL-5 cells have comparable negative control mutant fractions at the hprt locus (2.5 to 3 x 10⁻⁶). This comparison is presented in Figure 12. For all procarcinogens except Benzidine the MCL-5 cell line was significantly more sensitive that AHH-1 cells. The nitrosamines were more than 10,000 fold more active, Benzo(a)pyrene and Aflatoxin B₁ were about 1000 fold more active and 2-Acetoaminofluorene was 3 fold more active in MCL-5 cells. Only Benzidine gave comparable responses in both cell lines. Based on the mutagenic responses observed in MCL-5 cells and the comparisons to AHH-1 cells, the procarcinogen activating capacity has been substantially improved by the transfection procedure. MCL-5 cells were very sensitive to the mutagenic effects of two polycyclic aromatic hydrocarbons, two nitrosamines, a mycotoxin and an aromatic amide. These results indicate that this cell line can activate a spectrum of procarcinogens.

It should be noted that our understanding of human procarcinogen activation and the P450 forms responsible for this activation is still in its infancy. Therefore, it is reasonable to expect that additional P450s may be established to have primary responsibility for the activation of other procarcinogens. The development of the MCL-5 cell line establishes the feasibility of transfecting multiple human P450 cDNAs and it is reasonable to expect that additional P450s could be incorporated into the MCL-5 cells either in the same vectors or in an independently selected vector. Alternatively, a second cell line expressing a different set of P450 cDNAs could be developed.

One object of the invention was to construct a cell line expressing multiple P450 cDNAs and not to reconstruct the specific P450 profile of a particular tissue (i.e. liver). Accordingly, these results should not be interpreted as indicative of quantitative human tissue susceptabilty to procarcinogen exposure. However, as more is learned about specific P450 profiles in particular tissues, this study establishes that in principle it should be possible to reconstruct such a profile in a stable cell line for controlled analyses.

It should be understood that various changes and modifications of the preferred embodiments may be made within the scope of this invention. For example, other human cell lines or mammalian cell lines may be used to receive the recombinant expression vectors of the invention. Likewise, the methods of the invention may be used in connection with cloning human cytochrome P450 cDNA other than those described herein. Thus, it is intended that all matter contained in the above description shall be interpreted in an illustrative and not limiting sense.

### REFERENCES

1. Yoakum, G. H. (1984) Biotechniques 1/2, 24-30
2. Furth et al (1981) Anal. Biochem. 110: 1-8.
3. Young, R.A. et al (1983) Proc. Natl. Acad. Sci. USA 80: 1194-1198.
4. Young, R.A. et al (1983) Science 222: 778-782.
5. Sugden, B. et al (1985) Mol. Cell Biol. 5: 410-413.
6. Boyer, H.W. et al (1969) J. Mol. Biol. 41: 459-472.
7. Davies, R.L., Rudo, K., Turner, T.R. and Langenbach, R. (1989) Carcinogensis 10: 885-891.
8. Crespi, C.L., Langenbach, R. and Penman, B.W. (1990) Progress in Clinical and Biological Research. Mendelsohn, M.L. and Albertini, F.J. (eds) Wiley-Liss, NY, Vol. 340B, pp 97-106.
9. Phillips, I.R. et al (1985) Proc. Natl. Acad. Sci. USA 82: 983-987.
10. Davis et al (1980) Advanced Bacterial Genetics (Cold Spring Harbor Laboratory, Cold Spring Harbor, NY).
11. Maniatis. T. et al (1982) Molecular Cloning (Cold Spring Harbor Laboratory, Cold Spring Harbor, NY).
12. Denhardt, D.T. (1966) Biochem. Biophys. Res. Commun., 28: 641-651.
13. Southern, E.M. (1975) J. Mol. Biol. 98: 503-517.
14. Bolivar, F. et al (1977) Gene 2: 95-113.
15. Garger, S.J. et al (1983) Biochem. Biophys. Res. Commun. 117: 835-832.
16. Gonzalez, F.J. et al (1981) J. Biol. Chem. 256: 4697-4700.
17. McKnight, S.L. (1980) Nucl. Acids Res. 8: 5931-5948.
18. Crespi, C.L., Steimel, D.T., Aoyama, T., Gelboin, H.V. and Gonzalez, F.J. (1990) Mol. Carcinogenesis 3: 5-8.
19. Gonzalez, F.J., Schmid, B.J., Umeno, M., McBride, O.W., Hardwick, J.P., Meyer, U.A., Gelboin, H.V. and Idle, J.R. (1988) Human P450PCN1: sequence, chromosome, localization, and direct evidence through cDNA expression that P450PCN1 is nifedipine oxidase. DNA 7: 79-86.
20. Davies, R.L., Crespie, C.L., Rudo, K., Turner, T.R. and Langenbach, R. (1989) Development of a human cell line by selection and drug-metabolizing gene transfection with increased capacity to activate promutagens. Carcinocrenesis 10: 885-891.
21. Song B.J., Gelboin, H.V., Park, S.-S., Yang, C.S. and Gonzalez, F.J. (1986) J. Biol. Chem. 261: 16689-16697.
22. McKnight, S.L. (1980) Nucl. Acids Res. 8: 5949-5964.
23. Wagner et al (1981) Proc. Natl. Acad. Sci. USA 78: 1441-1445.
24. Crespi, C.L., Langenbach, R. and Penman, B.W. (1989) The development of a panel of human cell lines expressing specific human cytochrome P450 cDNAs. In: Mutation and the Environment, Mendelsohn, M.L. and Albertini, R. (Eds.) Alan Liss, NY, Vol. 340D, pp 97-106.
25. Greenlee, W.F. and Poland, A. (1978) J. Pharmacol. Exp. Ther. 205: 596-605.
26. Glatt, H. Kaltenbach, E. and Oesch, F. (1980) Cancer Res. 40: 2552-2556.
27. Burke, M.D. and Mayer, F.T. (1974) Drug Metab. Disp. 2: 583-588.
28. Crespi, D.L., Altman, J.D. and Marletta, M.A. (1985) Chem. Biol. Interact 53: 257-272.
29. Guenthner, T.M., Negishi, M. and Nebert, D.W. (1979) Anal. Biochem. 96: 201-207.
30. Peters, R., Bocher, R., Peaune, P., Iwasaki, M. and Gueugerich,, FP. (1990) In: Drugs, Metabolizing Enzymes: Genetics, Regulation and Toxicology. Ingelman-Sundberg, M. Gustafsson, J-A., and Orrenius, S. (eds). Proceedings of the VIIIth international symposium on Microsomes and Drug oxidations, Stockholm, Karolinska Institute,, 1990, Pg. 234.
31. Waxman, D.J., Ko, A. and Walsh, C. (1983) J. Biol. Chem. 258: 11937-11943.
32. Penman, B.W. and Crespi, C.L. (1987) Environ. Mol. Mutagen 10: 35-60.
33. Crespi, C.L., Penman, B.W., Leakey, J.A.E., Arlotto, M.P., Stark, A., Parkinson, A., Turner, T., Steimel, D.T., Rudo, K., Davies, R.L. and Langenbach, R. (1990) Carcinogenesis 11: 1293-1300.
34. Penman, B.W., Crespi, C.L., Komives, E.A., Liber, H.L. and Thilly, W.G. (1983) Mutat. Res. 108: 417-436.
35. Crespi, C.L., Seixas, G.M., Turner, T. and Penman, B.W. (1990) Environ. Molec. Mutagen. 15: 71-77.
36. Shimada, T. Mazrtin, M.V., Pruess-Schwartz, D. Marnett, L. and Guengerich, F.P. (1989) Cancer Res. 49: 6304-6312.
37. McManus, M.E., Burgess, W.M., Veronese, M.E., Huggett, A., Quattrochi, L.C., and Tukey, R.H. (1990) Cancer Res. 50: 3367-3376.
38. Skopek, T.R. et al (1978) Proc. Natl. Acad Sci. USA 75: 410-414.
39. Hoffman, G.R. et al (1975) Mutat. Res. 27: 307-318.
40. Chu E.H.Y. et al Proc. Natl. Acad. Sci. USA 61: 1306-1312.
41. Hsie, A.W., et al (1975) Somat. Cell Genet. 1: 383-389.

## Claims (Claims for the following Contracting State(s): AT, BE, CH/LI, DE, DK, FR, GB, IT, LU, NL, SE.)

1. An immortal mammalian cell line stably expressing at least four different transfected cytochrome P450s, at levels below that which renders P450 expression in the cell line unstable, wherein said P450s include P450IA2 and P450IIIA4.

2. A cell line as claimed in claim 1, wherein said P450s include P450IA2, P450IIE1 and P450IIIA4.

3. An immortal mammalian cell line stably expressing at least four different transfected cytochrome P450s, at levels below that which renders P450 expression in the cell line unstable, wherein said P450s include P450IIA3 and P450IIIA4.

4. A cell line as claimed in claim 3, wherein said P450s include P450IIA3, P450IIE1 and P450IIIA4.

5. A cell line as claimed in any of the preceding claims, wherein said cytochrome P450s include P450IA2, P450IIA3, P450IIE1 and P450IIIA4.

6. An immortal mammalian cell line as claimed in any of the preceding claims, transfected with plasmids pME23 as shown in figure 4 and pH441 as shown in figure 5.

7. A cell line as claimed in any of the preceding claims, wherein said cell line is a human cell line.

8. A cell line as claimed in any of the preceding claims, wherein said cytochrome P450s are human.

9. A method of conducting a metabolism, toxicity or mutagenicity assay, comprising using a cell line as claimed in any of claims 1-8.

10. A method as claimed in claim 9, the method comprising exposing a culture of a cell line as claimed in any of claims 1-8 to a test substance and assaying the metabolic, toxic and/or mutagenic effects, if any, of the substance on the cell line.

11. A method of conducting a mutagenicity assay as claimed in claim 10 comprising the steps of:
(a) exposing a culture of a cell line as claimed in any of claims 1-8 to the substance,
(b) detecting the number of mutant cells in the exposed culture, and
(c) comparing the frequency of mutation of the exposed cells to the frequency of mutation of unexposed cells to determine the mutagenicity of the substance.

## Claims (Claims for the following Contracting State(s): ES.)

1. A process for preparing an immortal mammalian cell line stably expressing at least four different transfected cytochrome P450s, at levels below that which renders P450 expression in the cell line unstable, the process comprising transfecting a mammalian cell line with plasmids pME23 as shown in figure 4 and pH441 as shown in figure 5.

2. A process as claimed in claim 1, wherein said cell line is a human cell line.

3. A method of conducting a metabolism, toxicity or mutagenicity assay, comprising using an immortal mammalian cell line stably expressing at least four different transfected cytochrome P450s, at levels below that which renders P450 expression in the cell line unstable, and transfected with plasmids pME23 as shown in figure 4 and pH441 as shown in figure 5.

4. A method as claimed in claim 3, the method comprising exposing a culture of an immortal mammalian cell line stably expressing at least four different transfected cytochrome P450s, at levels below that which renders P450 expression in the cell line unstable, and transfected with plasmids pME23 as shown in figure 4 and pH441 as shown in figure 5, to a test substance and assaying the metabolic, toxic and/or mutagenic effects, if any, of the substance on the cell line.

5. A method of conducting a mutagenicity assay as claimed in claim 4 comprising the steps of:
(a) exposing a culture of an immortal mammalian cell line stably expressing at least four different transfected cytochrome P450s, at levels below that which renders P450 expression in the cell line unstable, and transfected with plasmids pME23 as shown in figure 4 and pH441 as shown in figure 5 to the substance,
(b) detecting the number of mutant cells in the exposed culture, and
(c) comparing the frequency of mutation of the exposed cells to the frequency of mutation of unexposed cells to determine the mutagenicity of the substance.

## Patentansprüche (Patentansprüche für folgende(n) Vertragsstaat(en): AT, BE, CH/LI, de, DK, FR, GB, GR, IT, LU, NL, SE)

1. Unsterbliche Säugetier-Zellinie, die mindestens vier verschiedene transfizierte P950-Cytochrome unterhalb des Niveaus stabil exprimiert, das die P450-Expression in der Zellinie instabil macht, wobei die P450-Cytochrome P450IA2 und P450IIIA4 einschließen.

2. Zellinie nach Anspruch 1,wobei die P450-Cytochrome P450IA2, P450IIE1 und P450IIIA4 einschließen.

3. Unsterbliche Säugetier-Zellinie, die mindestens vier verschiedene transfizierte P450-Cytochrome unterhalb des Niveaus stabil exprimiert, das die P450-Expression in der Zellinie instabil macht, wobei die P450-Cytochrome P450IIA3 und P450IIIA4 einschließen.

4. Zellinie nach Anspruch drei, wobei die P450-Cytochrome P450IIA3, P450IIE1 und P450IIIA4 einschließen.

5. Zellinie nach einem der vorhergehenden Ansprüche, wobei die P450-Cytochrome P450IA2, P450IIA3, P450IIE1 und P450IIIA4 einschließen.

6. Unsterbliche Säugetier-Zellinie nach einem der vorhergehenden Ansprüche, transfiziert mit den Plasmiden pME23, wie aus Figur 4 ersichtlich, und pH441, wie aus Figur 5 ersichtlich.

7. Zellinie nach einem der vorhergehenden Ansprüche, wobei die Zellinie eine menschliche Zellinie ist.

8. Zellinie nach einem der vorhergehenden Ansprüche, wobei die P450-Cytochrome menschlich sind.

9. Verfahren zur Durchführung eines Stoffwechsel-, Toxizitäts- oder Mutagenitätstests; umfassend die Verwendung einer Zellinie nach einem der Ansprüche 1-8.

10. Verfahren nach Anspruch 9, wobei das Verfahren das Exponierung einer Kultur einer Zellinie nach einem der Ansprüche 1-8 gegenüber einer Testsubstanz und Untersuchen der metabolischen, toxischen und/oder mutagenen Wirkungen der Substanz, sofern vorhanden, auf die Zellinie umfaßt.

11. Verfahren zur Durchführung eines Mutagenitätstests nach Anspruch 10, umfassend die Schritte des:
(a) Exponierens einer Kultur einer Zellinie nach einem der Ansprüche 1-8 gegenüber der Substanz,
(b) Feststellens der Anzahl mutierter Zellen in der exponierten Kultur, und
(c) Vergleichens der Mutationshäufigkeit der exponierten Zellen mit der Mutationshäufigkeit von nicht-exponierten Zellen zur Ermittlung der Mutagenität der Substanz.

## Patentansprüche (Patentansprüche für folgende(n) Vertragsstaat(en): ES)

1. Verfahren zur Herstellung einer unsterblichen Säugetier-zellinie, die mindestens vier verschiedene transfizierte P450-Cytochrome unterhalb des Niveaus stabil exprimiert, das die P450-Expression in der Zellinie instabil macht, wobei das Verfahren das Transfizieren einer Säugetier-Zellinie mit den Plasmiden pME23, wie aus Figur 4 ersichtlich, und pH441, wie aus Figur 5 ersichtlich, umfaßt.

2. Verfahren nach Anspruch 1, wobei die Zellinie eine menschliche Zellinie ist.

3. Verfahren zur Durchführung eines Stoffwechsel-, Toxizitäts- oder Mutagenitätstests, umfassend die Verwendung einer unsterblichen Säugetier-Zellinie, die mindestens vier verschiedene transfizierte P450-Cytochrome unterhalb des Niveaus stabil exprimiert, das die P450-Expression in der Zellinie instabil macht, und die mit den Plasmiden pME23, wie aus Figur 4 ersichtlich, und pH441, wie aus Figur 5 ersichtlich, transfiziert ist.

4. Verfahren nach Anspruch 3, wobei das Verfahren das Exponieren einer Kultur einer unsterblichen Säugetier-Zellinie, die mindestens vier verschiedene transfizierte P450-Cytochrome unterhalb des Niveaus stabil exprimiert, das die P450-Expression in der Zellinie instabil macht, und die mit den Plasmiden pME23, wie aus Figur 4 ersichtlich, und pH441, wie aus Figur 5 ersichtlich, transfiziert ist, gegenüber einer Testsubstanz und das Untersuchen der metabolischen, toxischen und/oder mutagenen Wirkungen der Substanz, sofern vorhanden, auf die Zellinie umfaßt.

5. Verfahren zur Durchführung eines Mutagenitätstests nach Anspruch 4, umfassend die Schritte des:
(a) Exponierens einer Kultur einer unsterblichen Säugetier-Zellinie, die mindestens vier verschiedene transfizierte P450-Cytochrome unterhalb des Niveaus stabil exprimiert, das die P450-Expression in der Zellinie instabil macht, und die mit den Plasmiden pME23, wie aus Figur 4 ersichtlich, und pH441, wie aus Figur 5 ersichtlich, transfiziert ist, gegenüber der Substanz,
(b) Feststellens der Anzahl mutierter Zellen in der exponierten Kultur, und
(c) Vergleichens der Mutationshäufigkeit der exponierten Zellen mit der Mutationshäufigkeit von nicht-exponierten Zellen zur Ermittlung der Mutagenität der Substanz.

## Revendications (Revendications pour l'(les) Etat(s) contractant(s) suivant(s): AT, BE, CH/LI, DE, DK, FR, GB, GR, IT, LU, NL, SE)

1. Lignée cellulaire immortelle de mammifère, exprimant de manière stable au moins quatre cytochromes P450 transfectés différents, à des taux inférieurs à celui qui rend instable l'expression de P450 dans la lignée cellulaire, dans laquelle lesdits P450 comprennent P450IA2 et P450IIIA4.

2. Lignée cellulaire suivant la revendication 1, dans laquelle lesdits P450 comprennent P450IA2, P450IIE1 et P450IIIA4.

3. Lignée cellulaire immortelle de mammifère, exprimant de manière stable au moins quatre cytochromes P450 transfectés différents, à des taux inférieurs à celui qui rend instable l'expression de P450 dans la lignée cellulaire, dans laquelle lesdits P450 comprennent P450IIA3 et P450IIIA4.

4. Lignée cellulaire suivant la revendication 3, dans laquelle lesdits P450 comprennent P450IIA3, P450IIE1 et P450IIIA4.

5. Lignée cellulaire suivant l'une quelconque des revendications précédentes, dans laquelle lesdits cytochromes P450 comprennent P450IA2, P450IIA3, P450IIE1 et P450IIIA4.

6. Lignée cellulaire immortelle de mammifère suivant l'une quelconque des revendications précédentes, transfectée avec les plasmides pME23 représenté sur la figure 4 et pH441 représenté sur la figure 5.

7. Lignée cellulaire suivant l'une quelconque des revendications précédentes, ladite lignée cellulaire étant une lignée cellulaire humaine.

8. Lignée cellulaire suivant l'une quelconque des revendications précédentes, dans laquelle lesdits cytochromes P450 sont humains.

9. Méthode pour effectuer une analyse de métabolisme, de toxicité ou de mutagénicité, comprenant l'utilisation d'une lignée cellulaire suivant l'une quelconque des revendications 1 à 8.

10. Méthode suivant la revendication 9, la méthode comprenant l'exposition d'une culture d'une lignée cellulaire suivant l'une quelconque des revendications 1 à 8 à une substance d'essai et l'analyse des effets métaboliques, toxiques et/ou mutagènes, s'il en existe de quelconques, de la substance sur la lignée cellulaire.

11. Méthode pour effectuer une analyse de mutagénicité suivant la revendication 10, comprenant les étapes consistant :
(a) à exposer une culture d'une lignée cellulaire suivant l'une quelconque des revendications 1 à 8 à la substance,
(b) à détecter le nombre de cellules mutantes dans la culture exposée, et
(c) à comparer la fréquence de mutation des cellules exposées à la fréquence de mutation des cellules non exposées pour déterminer la mutagénicité de la substance.

## Revendications (Revendications pour l'(les) Etat(s) contractant(s) suivant(s): ES)

1. Procédé pour la préparation d'une lignée cellulaire immortelle de mammifère, exprimant de manière stable au moins quatre cytochromes P450 transfectés différents, à des taux inférieurs à celui qui rend instable l'expression de P450 dans la lignée cellulaire, procédé comprenant la transfection d'une lignée cellulaire de mammifère avec les plasmides pME23 représenté sur la figure 4 et pH441 représenté sur la figure 5.

2. Procédé suivant la revendication 1, dans lequel ladite lignée cellulaire est une lignée cellulaire humaine.

3. Méthode pour effectuer une analyse de métabolisme, de toxicité ou de mutagénicité, comprenant l'utilisation d'une lignée cellulaire immortelle de mammifère exprimant de manière stable au moins quatre cytochromes P450 transfectés différents, à des taux inférieurs à celui qui rend instable l'expression de P450 dans la lignée cellulaire, et transfectée avec les plasmides pME23 représenté sur la figure 4 et pH441 représenté sur la figure 5.

4. Méthode suivant la revendication 3, ladite méthode comprenant l'exposition d'une culture d'une lignée cellulaire immortelle de mammifère, exprimant de manière stable au moins quatre cytochromes P450 transfectés différents, à des taux inférieurs à celui qui rend instable l'expression de P450 dans la lignée cellulaire, et transfectée avec les plasmides pME23 représenté sur la figure 4 et pH441 représenté sur la figure 5, à une substance d'essai et l'analyse des effets métaboliques, toxiques et/ou mutagènes, s'il en existe de quelconques, de la substance sur la lignée cellulaire.

5. Méthode pour effectuer une analyse de mutagénicité suivant la revendication 4, comprenant les étapes consistant :
(a) à exposer une culture d'une lignée cellulaire immortelle de mammifère exprimant de manière stable au moins quatre cytochromes P450 transfectés différents, à des taux inférieurs à celui qui rend instable l'expression de P450 dans la lignée cellulaire, et transfectée avec les plasmides pME23 représenté sur la figure 4 et pH441 représenté sur la figure 5, à la substance,
(b) à détecter le nombre de cellules mutantes, dans la culture exposée, et
(c) à comparer la fréquence de mutation de cellules exposées à la fréquence de mutation de cellules non exposées pour déterminer la mutagénicité de la substance.
